# EUROPEAN PATENT APPLICATION

(11) **EP 4 180 061 A1**
(43) Date of publication of application: **17.05.2023**
(21) Application number: 21207536.0
(22) Date of filing: 10.11.2021
(51) Int. Cl.: A61K 47/68, A61P 35/00, C07D 498/14

(54) **ANTHRACYCLINE DERIVATIVE LINKER REAGENTS, ANTIBODY-DRUG CONJUGATES AND METHODS**

(71) Applicant: Nerviano Medical Sciences S.r.l., 20014 Nerviano (MI) (IT)
(72) Inventor: SALSA, Matteo, 28043 Bellinzago Novarese (NO) (IT); GASPARRI, Fabio, 20015 Parabiago (MI) (IT); ORSINI, Paolo, 20025 Legnano (MI) (IT); VALSASINA, Barbara, 20131 Milan (IT)

(57) **Abstract**

The present invention provides anthracycline-linker reagents for the preparation of therapeutic antibody-drug conjugate (ADC) compounds.

The present invention also provides therapeutic antibody-drug conjugate (ADC) compounds comprising anthracycline drug moieties, with biological activity against cancer cells. The compounds may inhibit tumor growth in mammals and may be useful for treating human cancer patients.

Aspects of the invention include methods of making, methods of preparing, methods of synthesis, methods of conjugation, and methods of purification of the anthracycline-linker reagents and of the antibody-drug conjugate compounds.

## Description

### Field of the invention

The present invention provides anthracycline-linker reagents for the preparation of therapeutic antibody-drug conjugate (ADC) compounds.

The present invention also provides therapeutic antibody-drug conjugate (ADC) compounds comprising anthracycline drug moieties, with biological activity against cancer cells. The compounds may inhibit tumor growth in mammals and may be useful for treating human cancer patients.

Aspects of the invention include methods of making, methods of preparing, methods of synthesis, methods of conjugation, and methods of purification of the drug-linker reagents and of the antibody-drug conjugate compounds.

### Background of the invention

Anthracyclines are antibiotic compounds that exhibit cytotoxic activity. Several studies have indicated that anthracyclines may operate to kill cells by a number of different mechanisms including: 1) intercalation with the DNA of a cell thereby inhibiting DNA-dependent nucleic acid synthesis; 2) production of free radicals which then react with cellular macromolecules to cause damage to the cells or 3) interactions with the cell membrane [see, e.g., C. Peterson et al., "Transport and storage of Anthracycline in experimental systems and human leukemia" in Anthracycline Antibiotics In Cancer Therapy (1982), pp.132-146; and N.R. Bachur, "Free Radical Damage" id. pp.97-102]. Because of their cytotoxic activity, anthracyclines have been used in the treatment of numerous cancers such as leukemia, breast carcinoma, lung carcinoma, ovarian adenocarcinoma and sarcomas [see e.g., P.H- Wiernik, in Anthracycline: Current Status And New Developments (1980), p. 11]. Commonly used anthracyclines include doxorubicin, epirubicin, idarubicin and daunomycin.

Morpholino analogs of doxorubicin and daunorubicin, formed by cyclization on the glycoside amino group, have greater potency (Acton et al (1984) J. Med. Chem. 638- 645; WO9802446; US 4464529; US 4672057; US 5304687). Although these compounds may be useful in the treatment of neoplasms and other disease states wherein a selected cell population is sought to be eliminated, their therapeutic efficacy is often limited by the dose-dependent toxicity associated with their administration.

Limitations in the use of anthracycline derivatives in clinical include inadequate accumulation in the tumor, cardiotoxicity, prolonged time of infusions, resistance of cancer cells overexpressing membrane transporters and dose-limiting side effects such as diarrhea and bone marrow suppression (Int. J. Nanomedicine, 2007; 2(4): 567-83; Med. Sci. Monit. 2000; 6(2); Adriamycin Review, pp 123-131, Mediokon, Belgium: European Press, 1975; Ann Intern Med. 1982; 96(2): 133-139).

Drug conjugation of cytotoxic drugs to molecules able to vehicle the drug thus improving tumor targeting or able to modify its pharmacokinetic properties is one of the strategies that has been undertaken to solve the above mentioned issues (Xie et al (2006) Expert. Opin. Biol. Ther. 6(3):281-291; Kovtun et al (2006) Cancer Res. 66(6):3214-3121. Different examples of conjugation of cytotoxic drugs (payloads) with proteins, peptides, aptamers, polymers or nanoparticles allowing better target delivery, improving solubility and in some cases other pharmacokinetic properties, such as increasing half-life or local concentration of the drug, and improving drug performances have been reported. As a matter of facts, the resultant conjugates have improved characteristics in terms of solubility, permeability into the cell, *in vivo* therapeutic window, controlled release, ability to reach the target according to the nature of the specific molecule conjugated with the cytotoxic agent, etc.

In this prospective, the conjugation of an antibody to a cytotoxic agent, thus generating antibody drug conjugates (ADCs) is one of the emerging approaches for the discovery of new drugs.

This approach incorporates the antibody specificity and cell killing activity of chemically conjugated highly cytotoxic agents. Antibody in ADC structure acts as a targeting agent and a nanoscale carrier to deliver a therapeutic dose of cytotoxic cargo into desired tumor cells.

Anthracycline derivative conjugates to antibody have been disclosed, for instance, in Int. Pat. App. WO2009/099741 and in WO2010/009124. Immunoconjugates and prodrugs of daunorubicin and doxorubicin have been prepared and studied (Kratz et al (2006) Current Med. Chem. 13:477-523; Jeffrey et al (2006) Bioorganic & Med. Chem. Letters 16:358-362. The antibody-drug conjugate BR96-doxorubicin reacts specifically with the tumor-associated antigen Lewis-Y and has been evaluated in phase I and II studies (Saleh et al (2000) J. Clin. Oncology 18:2282-2292; Ajani et al (2000) Cancer Jour. 6:78-81; Tolcher et al (1999) J. Clin. Oncology 17:478-484).

Hovever, ADCs as a potential targeted delivery system must be passed through all hurdles, including blood circulation, antigen binding, internalization, payload release, and eventual payload action. Off-target release of cytotoxic payloads is recognized as one of the issues that contribute to increase toxicity of such drugs, thus preventing their clinical development (Avicenna J Med Biotechnol. 2019 Jan-Mar; 11(1): 3-23. and K. R. Kozak (2020).

For this reason, there is an increasing demand for the development of functionalized cytotoxic agents suitable to be conjugated with different types of molecules that show a good balance between efficacy and toxicity.

We have now found that functionalized anthracycline derivatives, showing moderate cytotoxic activity when tested as such in the biological assay, when conjugated with an antibody, thus forming the correspondent ADCs, show unexpectedly a very good cytotoxic activity and very good safety profile. Therefore, the new ADC's can be useful to treat cancer and other pathologies.

### Definitions

"Anthracycline drug" or "Anthracycline derivative drug" is a compound of formula (II) endowed with cytotoxic activity. "Anthracycline-linker reagent" (Ant-L) is a compound comprising an anthracycline drug or anthracycline derivative drug covalently attached to a linker moiety that includes a reactive functional group. Anthracycline-linker reagent can be conveniently used to prepare the anthracycline-drug conjugates by reaction with a carrier moiety.

"Anthracycline derivative conjugate" or "anthracycline-drug conjugate" is a compound comprised of an anthracycline derivative drug covalently attached through a linker to a carrier moiety, including antibodies, proteins or peptides. Anthracycline derivative conjugate compounds include antibody-drug conjugate (ADC) compounds.

"Linker" or "link" is a chemical moiety comprising a covalent bond or a chain of atoms covalently attached to an Anthracycline drug" or "Anthracycline-drug derivative" (Ant) to form anthracycline-linker reagent (Ant-L).

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g., IgG, IgE, IgM, IgD, and IgA), class (e.g., IgG1, IgG2, IgG3, IgG4, IgA1 and IgA2) or subclass of immunoglobulin molecule. The immunoglobulins can be derived from any species, including human, murine, or rabbit origin.

An "ErbB receptor" is a receptor protein tyrosine kinase which belongs to the ErbB receptor family which are important mediators of cell growth, differentiation and survival. The ErbB receptor family includes four distinct members including epidermal growth factor receptor (EGFR, ErbB1, HER1), HER2 (ErbB2 or p185*^{neu}*), HER3 (ErbB3) and HER4 (ErbB4 or tyro2). The ErbB receptor will generally comprise an extracellular domain, which may bind an ErbB ligand; a lipophilic transmembrane domain; a conserved intracellular tyrosine kinase domain; and a carboxyl-terminal signaling domain harboring several tyrosine residues which can be phosphorylated. The ErbB receptor may be a "native sequence" ErbB receptor or an "aminoacid sequence variant" thereof. The ErbB receptor may be native sequence human ErbB receptor. Accordingly, a "member of the ErbB receptor family" is EGFR (ErbB1), ErbB2, ErbB3, ErbB4 or any other ErbB receptor currently known or to be identified in the future. Sequence identity screening has resulted in the identification of two other ErbB receptor family members; ErbB3 (US 5183884; US 5480968; Kraus et al (1989) PNAS (USA) 86:9193-9197) and ErbB4 (EP 599274; Plowman et al (1993) Proc. Natl. Acad. Sci. USA, 90:1746-1750; and Plowman et al (1993) Nature 366:473-475). Both of these receptors display increased expression on at least some breast cancer cell lines. Anti-ErbB2 antibodies have been characterized (US 5677171; US 5821337; US 6054297; US 6165464; US 6407213; US 6719971; US 6800738).

"Carrier moiety" is derived from polyclonal and monoclonal antibodies, proteins or peptides of natural or synthetic origin. Carrier moiety are suitable for conjugation with anthracycline-drug reagents of formula (I). Carrier moieties may be derived from polyclonal antibodies raised against tumor associated antigens; or from monoclonal antibodies binding to antigens preferentially or selectively expressed on tumor cell populations; or from natural or recombinant peptides or proteins or growth factors preferentially or selectively binding to tumor cells; or from natural or synthetic polymeric carriers such as polylysine, polyglutamic acid, polyaspartic acid and their analogues and derivatives, or such as dextran or other polymeric carbohydrate analogues and their derivatives; or from synthetic copolymers such as those derived from N-(2-hydroxypropyl)methacrylamide (HPMA) see: J. Kopecek, Macromolecules. H. Benoit & P. Rempp, Ed.: 505-520 (1982) Pergamon Press. Oxford, England; or from poly(aminoacid) copolymers such as poly(GIuNa, Ala, Tyr) which are useful as targetable drug-carriers for lung tissue R. Duncan et al., Journal of Bioactive and Compatible Polymers, Vol 4, July 1989.

The term payload, as used herein, refers to any chemical structure deriving from chemical or biochemical degradation of the antibody drug conjugate (ADC) comprising an anthracycline residue endowed with a cytotoxic activity. Particularly preferred are payloads of formula (II).

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies and is not to be construed as requiring production of the antibody by any particular method. For example, the monoclonal antibodies to be used in accordance with the present invention may be made by the hybridoma method first described by Kohler et al (1975) Nature 256:495 or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non-human primate (e.g., Old World Monkey or Ape) and human constant region sequences.

"Antibody fragments" comprise a portion of a full length antibody, generally the antigen binding or variable region thereof. Examples of antibody fragments include Fab, Fab', F(ab')₂, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "aminoacid side chain" includes those groups found in: (i) naturally occurring aminoacids such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine; (ii) minor aminoacids such as ornithine and citrulline; (iii) unnatural D-aminoacids, beta-aminoacids, synthetic analogs and derivatives of naturally occurring aminoacids; and (iv) all enantiomers, diastereomers, isomerically enriched, isotopically labelled, protected forms, and racemic mixtures thereof.

With the term "straight or branched C₁-C₆ alkyl" we intend any of the groups such as, for instance, methyl, ethyl, n-propyl, n-butyl, n-pentyl, isopropyl, n-butyl, isobutyl, sec-butyl, tert-butyl.

With the term "straight or branched C₁-C₄ hydroxyalkyl" we intend any of the groups such as, for instance, 2-hydroxyethyl, 3-hydroxypropyl, 2-hydroxypropyl, 4-hydroxybutyl, 3-hydroxybutyl, 2-hydroxybutyl.

With the term "halogen" we intend a fluorine, chlorine, bromine or iodine.

With the term "straight or branched C₁-C₄ haloalkyl", we intend any of the above defined C₁-C₄ alkyl groups which are substituted by one or more than one halogen atom such as, for instance, trifluoromethyl and trifluoroethyl.

The term "heterocyclyl" as used herein refers to a saturated or unsaturated non-aromatic 5- to 7- membered carbocyclic ring, wherein 1 to 3 carbon atoms are replaced by heteroatoms selected from nitrogen, oxygen and sulfur, wherein said heteroatoms may be directly connected to each other, nitrogen and sulfur may optionally be oxidized, nitrogen may optionally be quaternized or bring a substituent. Non limiting examples of heterocyclyl groups are, for instance, piperidinyl, piperazinyl, oxazolidinyl, 4-methylpiperazinyl, 4-ethylpiperazinyl, diazepinyl, etc.

The term "aryl" as used herein refers to carbocyclic hydrocarbons with from 1 to 2 ring moieties, either fused or linked to each other by single bonds, wherein at least one of the rings is aromatic. Examples of aryl groups according to the invention are, for instance, phenyl, biphenyl, α- or β-naphthyl, dihydronaphthyl, and the like.

The term "carbocycle" refers to 3- to 7-membered all-carbon monocyclic ring, which may contain one or more double bonds, but does not have a completely conjugated π-electron system.

Examples of carbocycles, without limitation, are cyclopropyl, cyclobutyl, cyclopentyl, cyclopentenyl, cyclohexanyl, cyclohexenyl and cyclohexadienyl.

The term "leaving group" refers to a group that can be substituted by another group in a substitution reaction. Such leaving groups are well-known in the art and examples include, but are not limited to, halides (fluoride, chloride, bromide and iodide), azides, sulfonates (e.g., an optionally substituted C₁-C₆ alkanesulfonate, such as methanesulfonate and trifluoromethanesulfonate, or an optionally substituted C₇-C₁₂ alkylbenzenesulfonate, such as p-toluenesulfonate), succinimide-N-oxide, p-nitrophenoxide, pentafluorophenoxide, tetrafluorophenoxide, carboxylates, aminocarboxylates (carbamates) and alkoxycarboxylates (carbonates). For substitutions at saturated carbon, halides and sulfonates are preferred leaving groups. For substitutions at a carbonyl carbon a halide, succinimide-N-oxide, p-nitrophenoxide, pentafluorophenoxide, tetrafluorophenoxide, a carboxylate, or an alkoxycarboxylate (carbonate) may for example be used as a leaving group. The term "leaving group" also refers to a group that is eliminated as a consequence of an elimination reaction, e.g., an electronic cascade reaction or a spirocyclization reaction. In this instance, an halide, a sulfonate, an azide, an aminocarboxylate (carbamate) or an alkoxycarboxylate (carbonate) may for example be used as a leaving group.

It is known to the person skilled in the art that transformation of a chemical functional group into another may require that one or more reactive centers in the compound containing such functional group have to be protected in order to avoid undesired side reactions. Protection of such reactive centers, and subsequent deprotection at the end of the synthetic transformations, can be accomplished following standard procedures described in the literature (see, for instance, Green, Theodora W. and Wuts, Peter G.M. - Protective Groups in Organic Synthesis, Third Edition, John Wiley & Sons Inc., New York (NY), 1999).

Therefore, the term "protecting group" refers to a group used to protect such reactive centers in a chemical synthesis, for example, a hydroxyl group (-OH), an amino group (-NH), a thiol group (-SH), a carbonyl group (-C=O), a carboxylic group (-COOH). Examples of protecting groups are those reported in the literature (see, for instance, ibidem).

The term "nitrogen protecting group" refers to a group that with the nitrogen atom form carbamates, amides, cyclic imides, *N*-alkyl and *N*-aryl amines. Such protecting groups are well-known in the art (see e.g. ibidem). Non limiting examples of carbamate protecting groups are, for instance, methyl and ethyl carbamate, 9-fluorenylmethyl carbamate (Fmoc), 2,2,2-trichloroethylcarbamate (Troc), *t*-butyl carbamate (BOC), vinyl carbamate (Voc), allyl carbamate (Alloc), benzyl carbamate (Cbz), p-nitrobenzyl and the like. Non limiting examples of amides are, for instance N-trichloroacetamide, *N*-trifluoroacetamide (TFA) and the like. Non limiting examples of cyclic imide protecting groups are, for instance, *N*-phthalimide, *N*-dithiasuccinoylimide (Dts) and the like. Non limiting examples of *N*-alkyl and *N*-aryl protecting groups are, for instance, *N*-allylamine, N-benzylamine and the like.

The term "hydroxyl protecting group" refers to a group that with the oxygen atom form ethers, esters, cyclic acetals or ketals. Such protecting groups are well-known in the art (see e.g. ibidem). Non limiting examples of ethers protecting groups are, for instance, alkyl ethers and benzyl ethers, such as methoxymethyl ether (MOM-OR), tetrahydropyranyl ether (THP-OR), allyl ether (Allyl-OR), benzyl ether (Bn-OR), triphenylmethyl ether (Tr-OR) and the like, or silyl ethers, such as trimethylsilyl ether (TMS-OR), *t*-butyldimethylsilyl ether (TBS-OR or TBDMS-OR), *t*-butyldiphenylsilyl ether (TBDPS-OR) diphenylmethylsilyl ether (DPMS-OR) and the like. Non limiting examples of esters protecting groups are, for instance, trifluoroacetate, benzoate (Bz-OR) and carbonates, such as ethylcarbonate and the like. Non limiting examples of cyclic acetals or ketals protecting groups are, for instance, methylene acetal, ethylidene acetal, methoxymethylene acetal and the like.

The term "active ester" refers to a functional group in which the alkoxy group of the ester moiety is a good leaving group. Examples of such alkoxy groups include, but are not limited to, succinimide-*N*-oxide (NHS esters), p-nitrophenoxide, pentafluorophenoxide, tetrafluorophenoxide, 1-hydroxybenzotriazole and 1-hydroxy-7-azabenzotriazole, and groups with comparable leaving capability. Unsubstituted alkyl-based alkoxy groups such as methoxy, ethoxy, isopropoxy, and t-butoxy do not qualify as good leaving groups and methyl, ethyl, isopropyl, and t-butyl esters are therefore not considered to be active esters.

The term "electron withdrawing group" refers to an atom group that draws electron density from neighboring atoms towards itself, usually by resonance or inductive effects. Non limiting examples are halogens, trifluoromethyl group, nitro (NO₂) group and nitrile (CN).

The term "nucleophiles" refers to molecules that bear a nucleophilic group. The term "nucleophilic group" refers to a species that donates an electron-pair to an electrophilic group to form a chemical bond in a chemical reaction. Examples of such nucleophilic groups include, but are not limited to halogens, amines, nitrites, azides, hydroxyls, alkoxyde anions, carboxylate anions, thiols, thiolates, etc.

The term "electrophilic group" refers to a species that accepts an electron-pair from a nucleophilic group to form a chemical bond in a chemical reaction. Examples of such electrophilic groups include, but are not limited to esters, aldehydes, amides, ketons, etc.

The term "unnatural D-aminoacid" refers to the D-stereoisomer of the naturally occurring aminoacid (L-stereoisomer).

### Summary of the invention

First object of the present invention is to provide an anthracycline-linker reagent of formula (I), comprising an anthracycline drug (Ant) covalently attached to a linker (L)

Ant-L (I)

wherein:

Ant is an anthracycline drug moiety of formula (II): 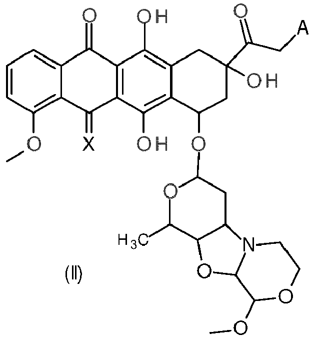 wherein:
**X** is O or NH and
**A** is a heterocycle (Het) or carbocycle (Cb) selected from the group consisting of (IIIa)-(IIIe): 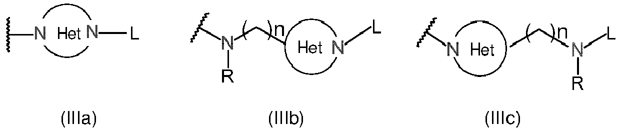 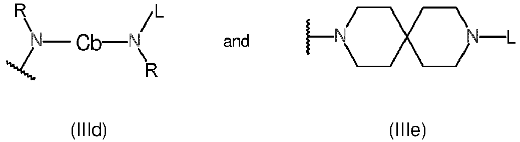 wherein:
   **R** is independently hydrogen, or a straight or branched (C₁-C₆) alkyl;
   **n** is an integer from 0 to 6;
**L** is a linker of formula (VI):

   W-PE-RM (VI)

   wherein:
**W** is independently null or a self immolative system selected from the group consisting of (Va)-(Vc): 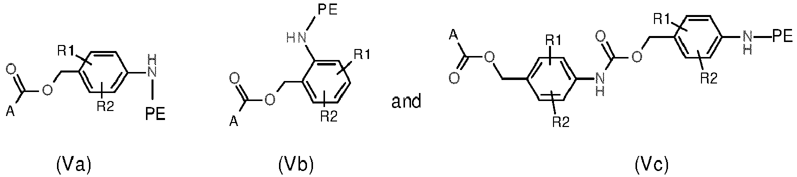 wherein:
   **R1** and **R2** are, each independently, hydrogen, halogen, methyl, ethyl or straight or branched C₁-C₄ hydroxyalkyl;
**PE** is independently null or a dipeptidic or tripeptidic moiety, consisting of any combination of natural L-aminoacids and unnatural D-aminoacids.
**RM** is a reactive moiety selected from the group consisting of (Vla)-(Vlc): 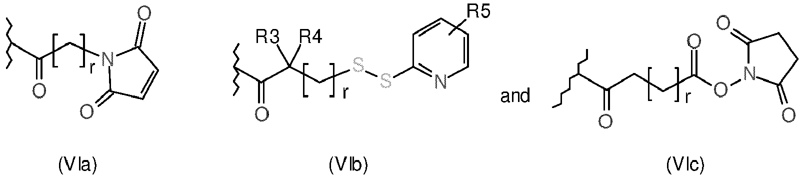 wherein:
   **R3** and **R4** are, each independently, hydrogen, halogen, methyl, ethyl, straight or branched C₁-C₄ hydroxyalkyl, straight or branched C₁-C₄ haloalkyl, or R3 and R4 taken together form a 3- to 6-membered carbocycle;
   **R5** is hydrogen, C₁-C₃ alkyl or an electron-withdrawing group, comprising NO₂ or CN group; r is an integer from 0 to 7.

In the compound of formula (I) the self immolative system **W** tethers in a stable way the moiety **A** of the anthracycline derivative drug of formula (II) to the **PE-RM** residue, or to the **RM** moiety when **PE** is null.

The linkage between **RM** and the connecting atoms of groups **PE;** or **W,** when **PE** is null; or **A,** when **W** and **PE** are both null, may form an amide, ester or thioester group.

Preferred anthracycline-linker reagent of formula (I) are the compounds wherein:
**A** is a heterocyclic or carbocyclic group selected from the group consisting of: 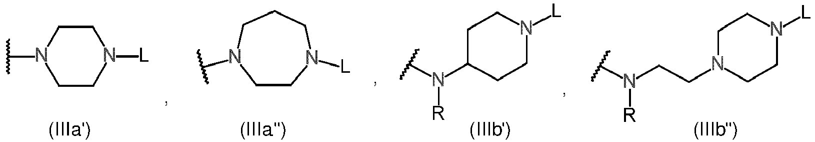 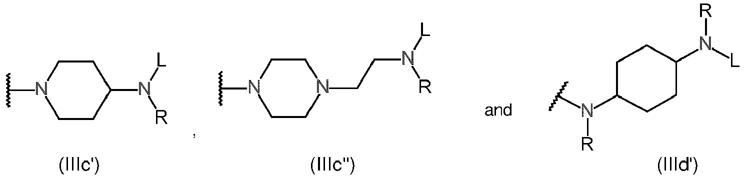 wherein:
   **R** is a straight or branched (C₁-C₆) alkyl;
L is a linker of formula (VI) wherein **W** is a group (Va') or (Vb'): 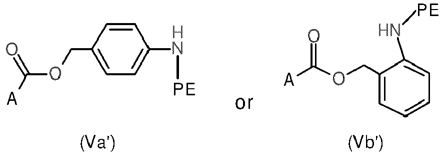
**PE** is a dipeptide or tripeptide moiety, consisting of any combination of the following aminoacids: glycine, alanine, leucine, valine, citrulline, phenylalanine, wherein the C-terminal aminoacid residue is linked to **W,** and the N-terminal aminoacid residue is linked to **RM.**
**RM** is a group selected from (Vla)-(Vlc): 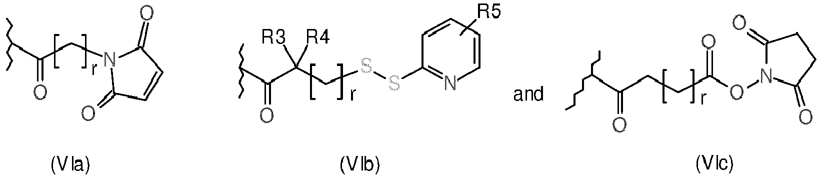 wherein:
   **R3, R4** and **R5** are hydrogen;
   **r** is an integer preferably from 3 to 5.

More preferred anthracycline-linker reagent of formula (I) are the compounds wherein:
**A** is a heterocyclic group selected from the group consisting of (IIIa'), (IIIa"), (IIIb') and (IIIc');
   wherein:
   **R** is a straight (C₁-C₆) alkyl;
**W** is a group (Va') or (Vb'):
   **PE** is a dipeptide selected from valine-alanine and valine-citrulline or a tripeptide phenylalanine-leucine-glycine wherein:
   the C-terminal aminoacid residue is linked to **W** and the N-terminal aminoacid residue is linked to **RM;**
**RM** is a reactive moiety selected from the group (Vla)-(Vlc):
   wherein:
   **R3, R4** and **R5** are hydrogen, and
   **r** is an integer from 3 to 5;

Other more preferred anthracycline-linker reagent of formula (I) are the compounds wherein:
**A** is a heterocyclic group selected from the group consisting of (IIIa'), (IIIa"), (IIIb') and (IIIc');
   wherein:
   **R** is a straight (C₁-C₆) alkyl; and
L is a linker selected from the group consisting of (IVa)-(IVc): 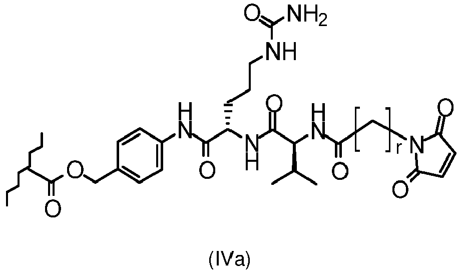 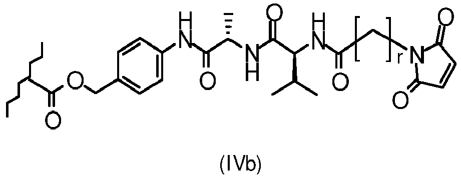 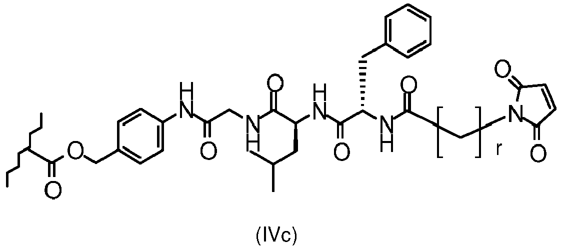 wherein:
   **r** is an integer from 3 to 5.

Preferred specific anthracycline-linker reagent compounds of formula (I), or a pharmaceutically acceptable salt thereof, are the compounds listed below:
[4-[[(2S)-2-[[(2S)-2-[6-(2,5-dioxopyrrol-1-yl)hexanoylamino]-3-methyl-butanoyl]amino]-5-ureido-pentanoyl]amino]phenyl]methyl 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate (comp 1);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7jtridecan-4-yl]oxy]-11-oxo-3,4-dihydro-1H-tetracen-2-yl]ethyl]amino]piperidine-1-carboxylate (comp 2);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]piperazine-1-carboxylate (comp 3);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11 - trioxa-1-azatricyclo[7.4.0.02,7]tridecan-4-yl]oxy]-11-oxo-3,4-dihydro-1H-tetracen-2-yl]ethyl]piperazine-1-carboxylate (comp 4);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl N-methyl-N-[1-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-4-piperidyl]carbamate (comp 5);
[4-[[(2S)-2-[[(2S)-2-[6-(2,5-dioxopyrrol-1-yl)hexanoylamino]-3-methyl-butanoyl]amino]propanoyl]amino]phenyl]methyl N-methyl-N-[1-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-4-piperidyl]carbamate (comp 6);
[4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl 4-[methyl-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]amino]piperidine-1-carboxylate (comp 7);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[methyl-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]amino]piperidine-1-carboxylate (comp 8);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyc!o[7.4.0.02,7]tridecan-4-yl]oxy]-6,11-dioxo-3,4-dihydro-1H-tetracen-2-yl]ethyl]piperazine-1-carboxylate (comp 9);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1 H-tetracen-2-yl]ethyl]piperazine-1-carboxylate (comp 10);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl N-methyl-N-[1-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl]carbamate (comp 11);
[4-[[(2S)-2-[[(2S)-2-[6-(2,5-dioxopyrrol-1-yl)hexanoylamino]-3-methyl-butanoyl]amino]propanoyl]amino]phenyl]methyl N-methyl-N-[1-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl] carbamate (comp 12);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7jtridecan-4-yl]oxy]-11-oxo-3,4-dihydro-1H-tetracen-2-yl]ethyl]-1,4-diazepane-1-carboxylate (comp 13);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-1,4-diazepane-1-carboxylate (comp 14);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-1,4-diazepane-1-carboxylate (comp 15) and
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1 - azatricyclo[7.4.0.02,7]tridecan-4-yl]oxy]-6,11-dioxo-3,4-dihydro-1H-tetracen-2-yl]ethyl]-1,4-diazepane-1-carboxylate (comp 16).

In the anthracycline-linker reagent of formula (I) the bond between **Ant** and the linker **L,** or one or more of the covalent bonds between **W** and **PE** or **PE** and **RM** of the linker itself, may be stable outside a cell, i.e. extracellular, or it may be cleavable by enzymatic activity, hydrolysis, or other metabolic conditions thus releasing an anthracycline payload that is the compound exhibiting cytotoxic activity.

It is clear from the people skill in the art that when a bound is broken the valence of the involved atoms is restored with hydrogen atom or with OH group depending upon the breaking type reaction. Therefore, without limiting the scope of the present invention, when in a compound of formula (I) the **Ant-L** bond is broken the released anthracycline payload is a compound of formula (II) wherein,
**A** is a heterocyclic or carbocyclic group selected from the group consisting of: 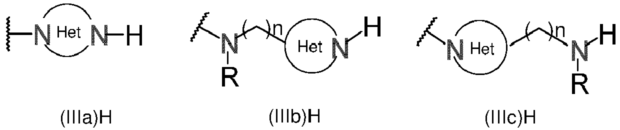 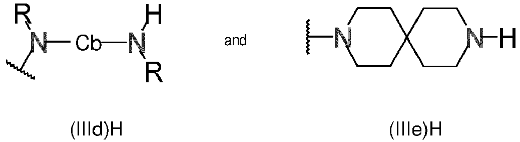 wherein:
**Het, Cb, R** and **n** are as defined above.

The anthracycline-linker reagent of formula (I) are compound comprising a reactive functionality (RM moiety) that can react with a carrier moiety thus forming an anthracycline drug conjugate. When the carrier moiety is an antibody (Ab) the conjugate is an Antibody-drug conjugates (ADC).

Second object of the present invention is to provide Antibody-drug conjugates (ADCs) of formula (Ia): 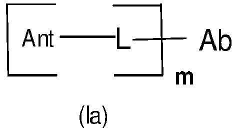 wherein:
**Ab** includes any unit, type, or class of antibody that binds or reactively associates or complexes with a receptor, antigen or other receptive moiety associated with a given target-cell population;
**m** (drug loading) is 1, 2, 3, 4, 5, 6, 7 or 8;
**Ant-L** is an anthracycline-linker reagent of formula (I) wherein the **RM** moiety of the linker **L** reacts with a cysteine thiol, or an amine, e.g. N- terminus or aminoacid side chain such as lysine, of the antibody (Ab) thus forming the antibody drug conjugate of formula (Ia). For clarity, the linkage between **RM** and the antibody **Ab**, thus forming the antibody-drug conjugate of formula (Ia), is reported below: 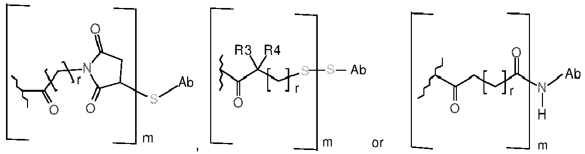 wherein:
   the broken line represent the attachment point of **RM** residue to **PE;** or to **W,** when **PE** is null; or to **A** of the anthracycline drug of formula (II), when both **W** and **PE** are null;
   **R3** and **R4** are as defined above;
   **r** is an integer from 0 to 7; and
   **m** is as defined above.

The anthracycline drug to antibody ratio, or drug loading, is represented by **m** for formula (Ia) compounds. The drug loading value **m** is 1 to 8. Formula (Ia) compounds include all mixtures of variously loaded and attached antibody-drug conjugates where 1, 2, 3, 4, 5, 6, 7 or 8 drug moieties are covalently attached to the antibody; preferably **m** is 1, 2, 3 or 4.

Preferred ADCs of formula (Ia) are the compounds wherein:
**Ant-L** is an anthracycline-linker reagent selected from the compounds 1 to 16, and
**m** and **Ab** are as defined above.

More preferred ADCs of formula (Ia) are the compounds wherein:
**Ab** is an antibody which binds to one or more tumor-associated ErbB2 antigens or cell-surface ErbB2 receptor and
**Ant-L** and **m** are as defined above.

Another more preferred ADCs of formula (Ia) are the compounds wherein:
**Ab** is Trastuzumab (TRZ)

Most preferred ADC of formula (Ia) are the compounds selected from the group consisting of compounds (Ia^{I}) - (Ia^{XVI}): 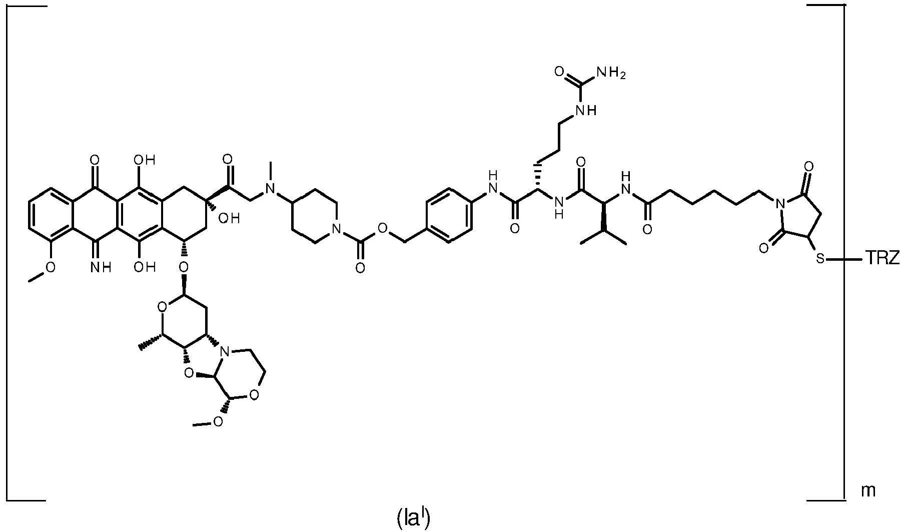 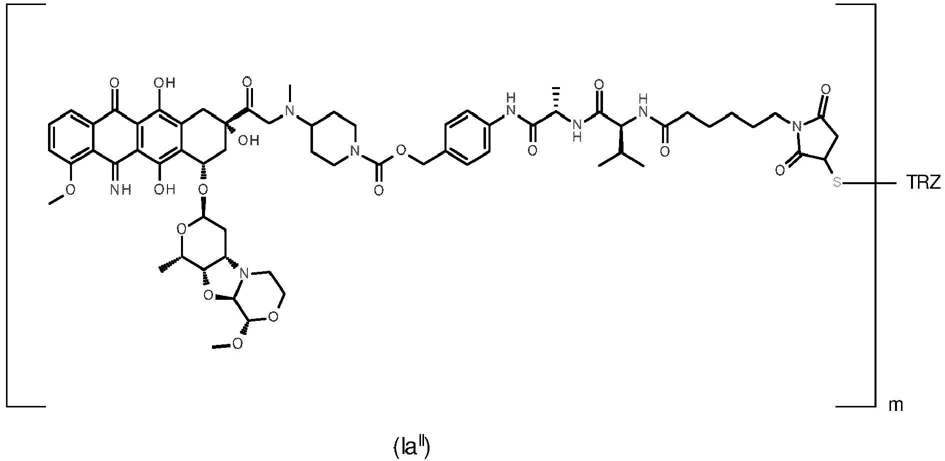 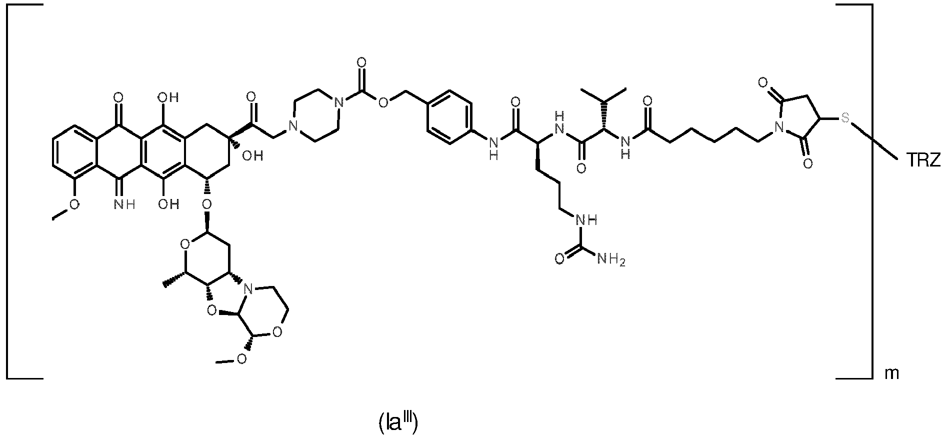 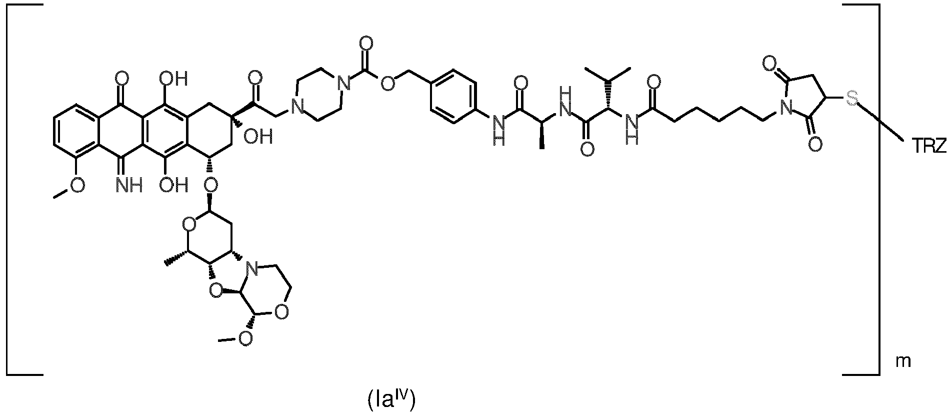 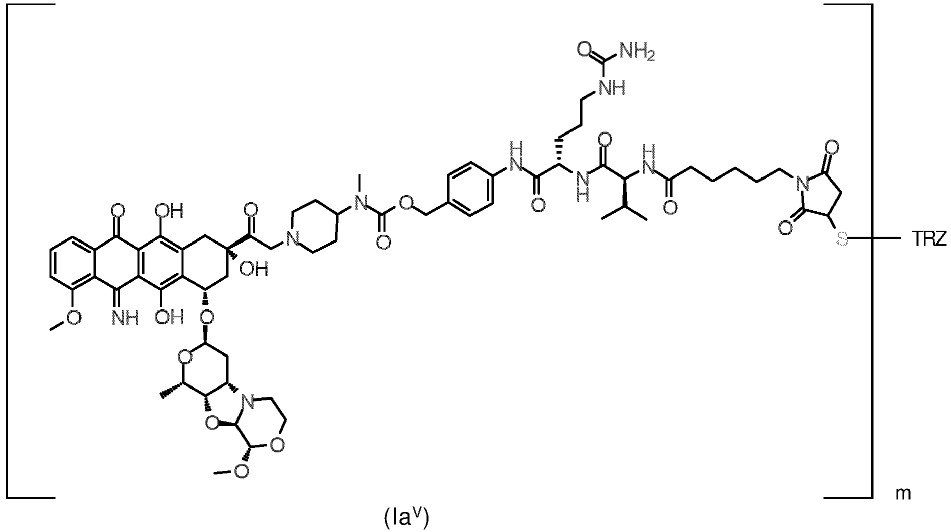 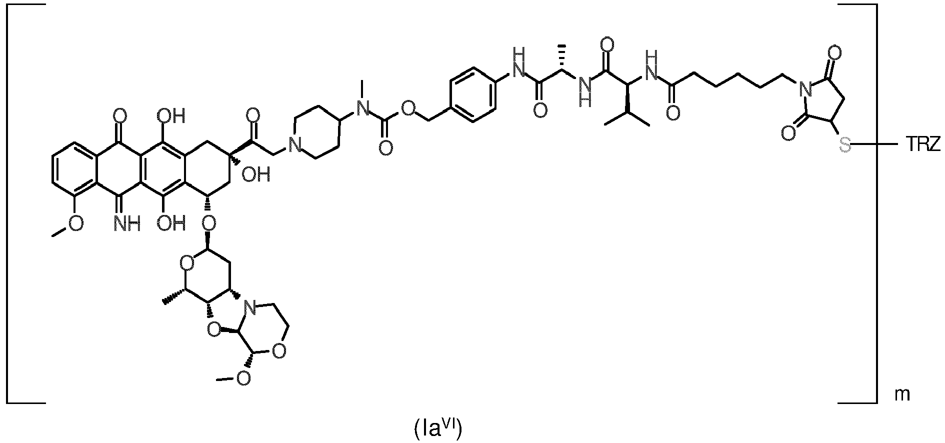 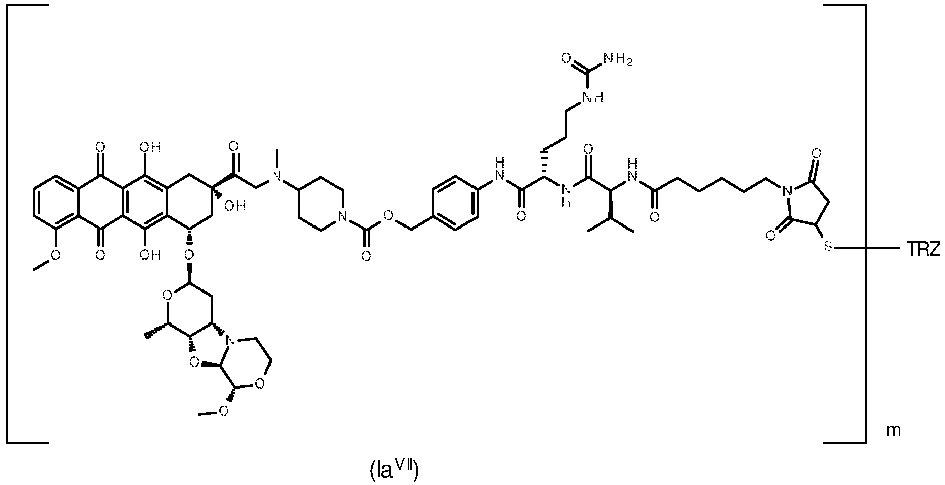 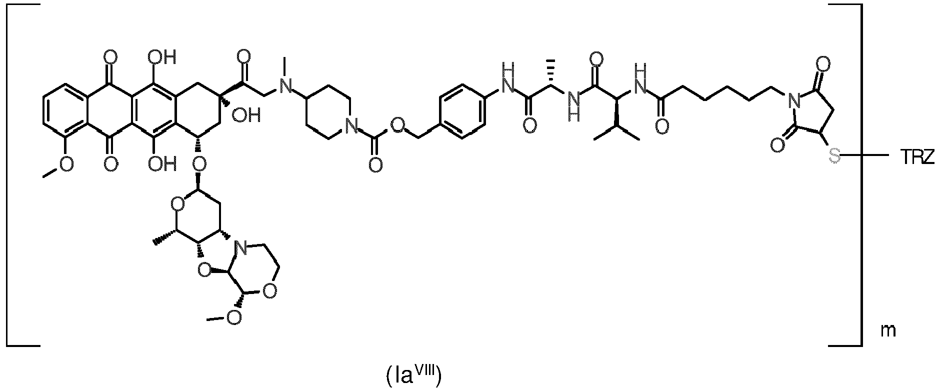 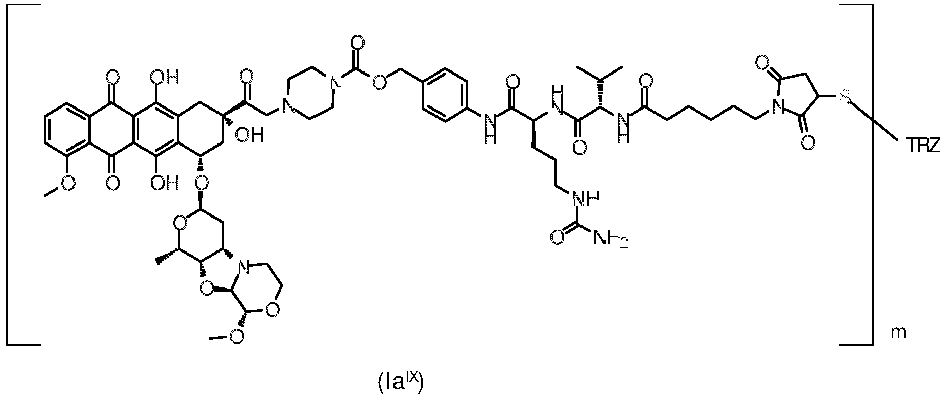 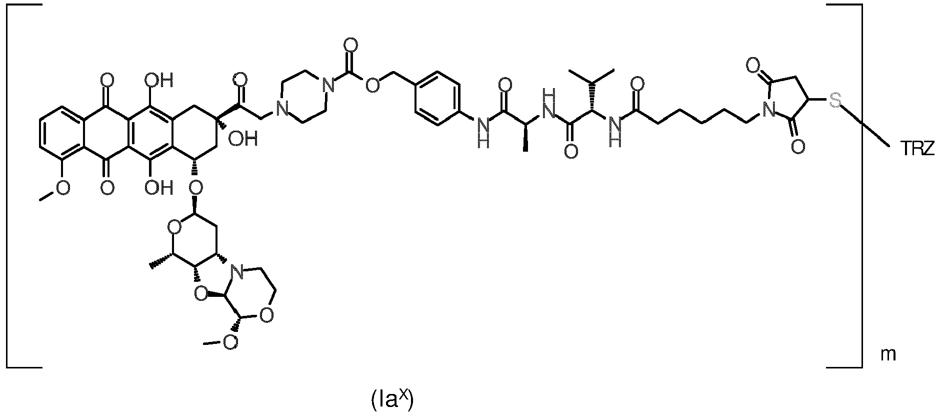 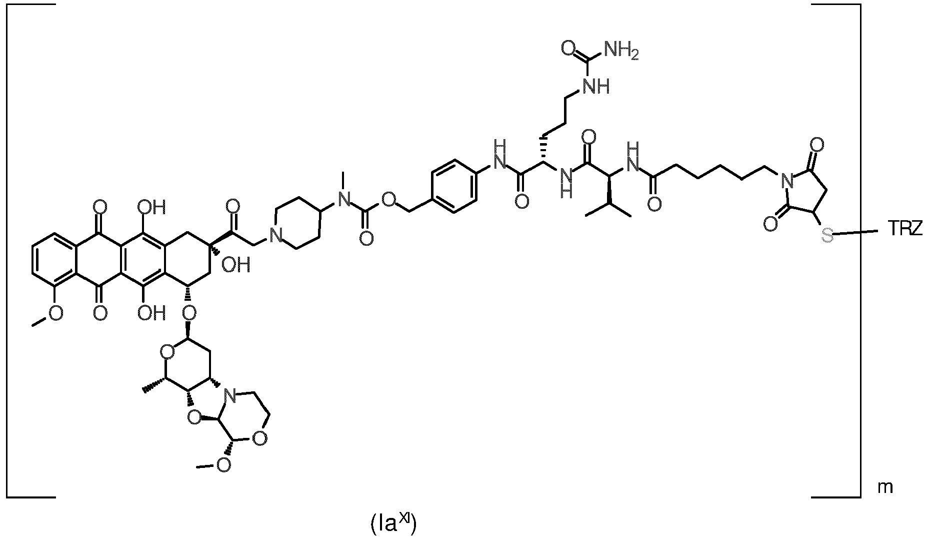 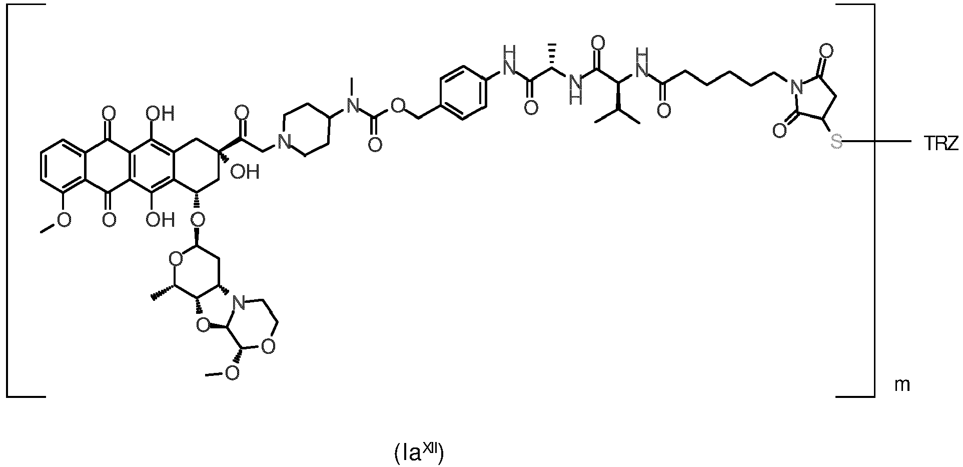 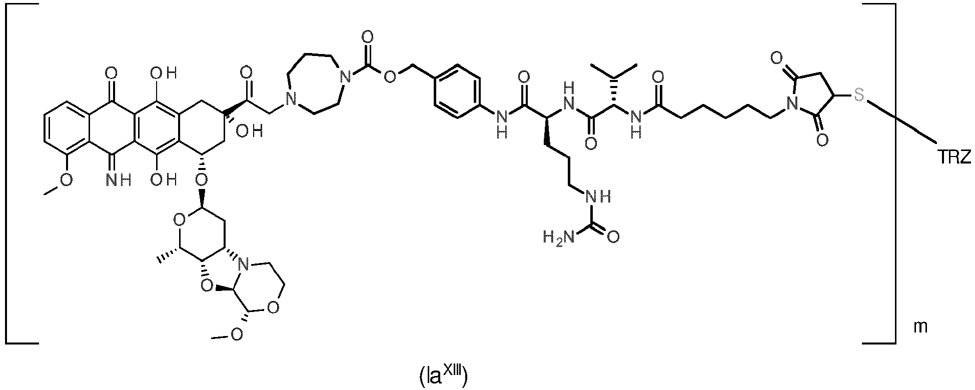 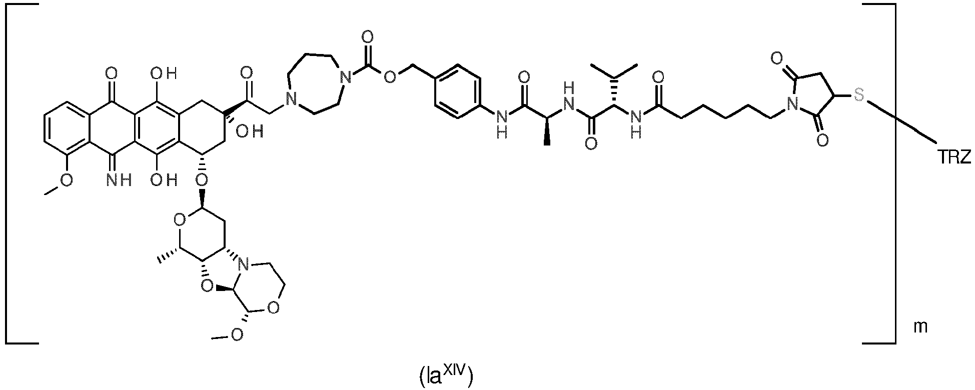 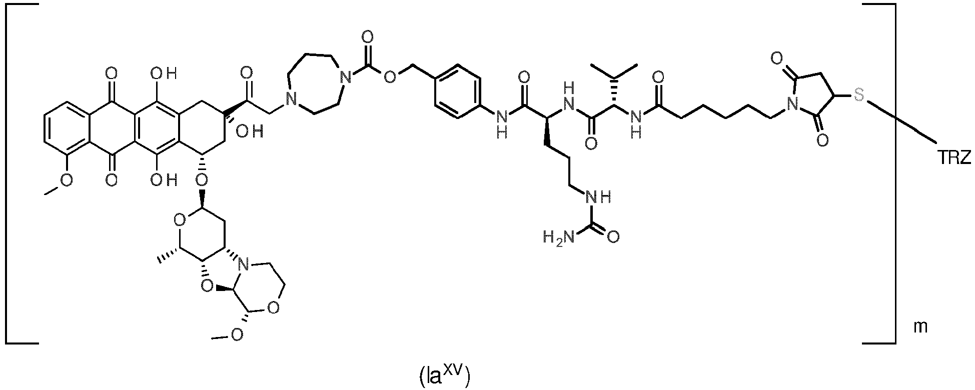 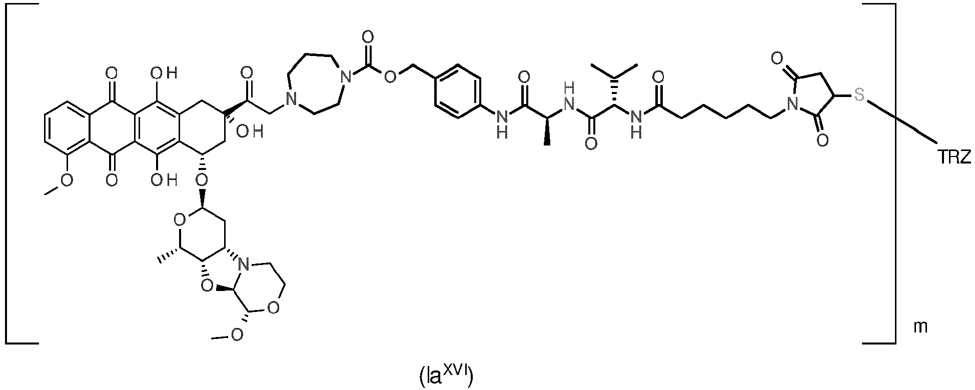 wherein m is 1, 2, 3, 4, 5, 6, 7 or 8. Preferably **m** is 1, 2, 3 or 4.

**Ant-L** bond and/or **L-Ab** bond and/or the covalent bonds connecting the moiety W, PE and RM of the linker (L) may be stable outside a cell, i.e. extracellular, or it may be cleavable by enzymatic activity, hydrolysis, or other metabolic conditions.

The bonds are preferably stable extracellularly. Before transport or delivery into a cell, the antibody-drug conjugate (ADC) is preferably stable and remains intact, i.e. the antibody remains linked to the drug moiety. The linkers are stable outside the target cell and may be cleaved at some efficacious rate inside the cell. An effective linker will: (i) maintain the specific binding properties of the antibody; (ii) allow intracellular delivery of the conjugate or the anthracycline payload; (iii) remain stable and intact, i.e. not cleaved, until the conjugate has been delivered or transported to its targeted site; and (iv) maintain a cytotoxic, cell- killing effect or a cytostatic effect of the anthracycline payload. Stability of the ADC may be measured by standard analytical techniques such as mass spectroscopy, HPLC, and the separation/analysis technique LC/MS.

ADCs of the present invention as well as anthracycline-drug of formula (I) are new compounds characterized in that they comprise a moiety (A) enclosing two secondary or tertiary amine functions that heavily affect, among other parameters, polarity and solubility of the molecules and therefore their pharmacokinetic properties. It can be speculated that, if unspecific release of the payload from the ADC occurs, the presence of protonable moieties on the payload structure, as in the compounds of the present invention, can prevent passive diffusion into cells of the released cytotoxic payload, thus reducing unspecific toxicity and improving the safety profile of the ADC.

The peculiarity of new (A) moiety in the structure of the ADC strongly differentiates the structure of the present invention from that of the prior arts both from a chemical structural point of view and from the chemical physical characteristics.

### DRUG LOADING

The drug loading is represented by **m** in an antibody-drug conjugate molecule of formula (Ia). Drug loading may range from 1 to 8 anthracycline drugs (Ant) per antibody (Ab), i.e. where **1,** 2, 3, 4, 5, 6, 7 or 8 anthracycline drug moieties are covalently attached to the antibody through a linker (L); preferably **m** is 1 2 3 or 4. The average number of anthracycline drugs per antibody in preparations of ADC from conjugation reactions may be characterized by conventional means such as mass spectroscopy, ELISA assay, electrophoresis, and HPLC. The quantitative distribution of ADC in terms of **m** may also be determined. By ELISA, the averaged value of **m** in a particular preparation of ADC may be determined (Hamblett et al (2004) Clin. Cancer Res. 10:7063-7070; Sanderson et al (2005) Clin. Cancer Res. 11:843-852). However, the distribution of **m** (anthracycline drug) values is not discernible by the antibody-antigen binding and detection limitation of ELISA. Also, ELISA assay for detection of antibody-drug conjugates does not determine where the anthracycline drug moieties are attached to the antibody, such as the heavy chain or light chain fragments, or the particular aminoacid residues. In some instances, separation, purification, and characterization of homogeneous ADC where **m** is a certain value from ADC with other drug loadings may be achieved by means such as reverse phase HPLC or electrophoresis.

For some antibody-drug conjugates, **m** may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.

Typically, fewer than the theoretical maximum of drug moieties are conjugated to an antibody during a conjugation reaction. An antibody may contain, for example, many lysine residues that do not react with the anthracycline-linker reagents (Ant-L). Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. Additionally, the antibody must be subjected to denaturing conditions to reveal reactive nucleophilic groups such as lysine or cysteine. The loading (drug/antibody ratio) of an ADC may be controlled in several different manners, including: (i) limiting the molar excess of anthracycline-linker reagents (Ant-L), (ii) limiting the conjugation reaction time or temperature, and (iii) partial or limiting reductive conditions for cysteine thiol modification.

Cysteine amino acids may be engineered at reactive sites in an antibody and which do not form intrachain or intermolecular disulfide linkages (US 7521541). The engineered cysteine thiols may react with anthracycline-linker reagents (Ant-L) of the present invention which have thiol-reactive, electrophilic groups such as compound of formula (VIa) or (Vlb) to form ADC with cysteine engineered antibodies and the anthracycline derivative drug moieties. The location of the drug moiety can thus be designed, controlled, and known. The drug loading can be controlled since the engineered cysteine thiol groups typically react with an anthracycline-linker reagent in high yield. Engineering an IgG antibody to introduce a cysteine amino acid by substitution at a single site on the heavy or light chain gives two new cysteines on the symmetrical antibody. A drug loading near 2 can be achieved and near homogeneity of the conjugation product ADC.

Where more than one nucleophilic or electrophilic group of the antibody reacts with anthracycline-linker reagents (Ant-L), then the resulting product is a mixture of ADC compounds with a distribution of anthracycline drug moieties attached to an antibody, e.g. 1, 2, 3, etc. Liquid chromatography methods such as polymeric reverse phase (PLRP) and hydrophobic interaction (HIC) may separate compounds in the mixture by drug loading value. Preparations of ADC with a single drug loading value m may be isolated, however, these single loading value ADCs may still be heterogeneous mixtures because the drug moieties may be attached, via the linker, at different sites on the antibody. Thus, the ADC compositions of the invention include mixtures of ADC compounds where the antibody has one or more anthracycline derivative drug moieties and where the anthracycline drug moieties may be attached to the antibody at various aminoacid residues.

The conjugates of formula (Ia) of the present invention are useful therapeutic agents since they contain cleavable bonds, which releases the anthracycline payload upon hydrolysis or "in vivo" enzymatic cleavage.

It is well known that in malignant tumors there is a high rate of glycolysis compared to normal tissue. This causes an increase in the production of lactate and thus a decrease of the pH in the tumor see: H. M. Rauen et al., Z. Naturforsch, Teil B, 23 (1968) 1461. The invention affords a two level specificity of action of the compounds, the first one consisting in a preferential localization of the conjugate in the tumor tissue by means of antigenic recognition, and the second one consisting in a preferential release of the drug in its active form in the tumor tissue by means of preferential acidic cleavage. While not limiting the scope or utility of the compositions or methods of the invention, the linkers described herein may be cleaved in vivo under localized or systemic acidic conditions, thus separating the targeting antibody from the Anthracycline payload.

The present invention also provides methods of synthesizing the anthracycline conjugates of formula (Ia) and anthracycline drug reagents (I), prepared through a process consisting of standard synthetic transformations, and their isomers, tautomers, hydrates, solvates, complexes, metabolites and N-oxides.

Moreover, the present invention provides a pharmaceutical composition comprising a therapeutically effective amount of a derivative of formula (Ia) or a pharmaceutically acceptable salt thereof as defined above and at least one pharmaceutically acceptable excipient, carrier or diluent.

The pharmaceutical compositions containing the compounds of the invention are usually prepared following conventional methods and are administered in a suitable pharmaceutical form. For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gum, gelatine methylcellulose, carboxymethylcellulose or polyvinyl pyrrolidone; disintegrating agents, e.g. starch, alginic acid, alginates or sodium starch glycolate; effervescing mixtures; dyestuffs; sweeteners; wetting agents, such as lecithin, polysorbates, laurylsulfates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, for instance, syrups, emulsions and suspensions. As an example, the syrups may contain, as carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusions may contain, as a carrier, sterile water or preferably they may be in the form of sterile, aqueous, isotonic, saline solutions or they may contain propylene glycol as a carrier. The suppositories may contain, together with the active compound, a pharmaceutically acceptable carrier, e.g. cocoa butter, polyethylene glycol, a polyoxyethylene sorbitan fatty acid ester surfactant or lecithin.

The present invention further provides a pharmaceutical composition comprising a therapeutically effective amount of a derivative of formula (Ia) and one or more chemotherapeutic agents.

The present invention also provides a pharmaceutical composition comprising a therapeutically effective amount of a derivative of formula (Ia) in combination with known anticancer treatments such as radiation therapy or chemotherapy regimen, in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER2 agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

Additionally, the invention provides a product comprising a derivative of formula (Ia) or a pharmaceutically acceptable salt thereof, as defined above, and one or more chemotherapeutic agents, as a combined preparation for simultaneous, separate or sequential use in anticancer therapy.

If formulated as a fixed dose, such combination products employ the compounds of this invention within the dosage range described below and the other pharmaceutically active agent within the approved dosage range.

Compounds of formula (Ia) may be used sequentially with known anticancer agents when a combination formulation is inappropriate.

The compounds of formula (Ia) of the present invention, suitable for administration to a mammal, e.g. to humans, can be administered by the usual routes and the dosage level depends upon the age, the weight, the conditions of the patient and the administration route.

For example, a suitable dosage adopted for iv administration of a compound of formula (Ia) may range from about 0.1 to about 10 mg/kg per dose daily. The compounds of the invention can be administered in a variety of dosage forms, e.g. orally, in the form of tablets, capsules, sugar or film coated tablets, liquid solutions or suspensions; rectally in the form of suppositories; parenterally, e.g. subcutaneously, intramuscularly, or through intravenous and/or intrathecal and/or intraspinal injection or infusion.

In yet another aspect the invention provides a derivative of formula (Ia) or a pharmaceutically acceptable salt thereof, as defined above, for use as a medicament.

The present invention also provides a derivative of formula (Ia) as defined above, for use in a method of treating cancer, cellular proliferation disorders and viral infections.

Preferably, a derivative of formula (Ia) as defined above, is for use in a method of treating specific types of cancers, such as but not limited to: carcinomas, including bladder, breast, colon, kidney, liver, lung, comprising small cell lung cancer, esophagus, gall-bladder, ovary, pancreas, stomach, cervix, thyroid, prostate, and skin carcinoma, comprising squamous cell carcinoma; hematopoietic tumors of lymphoid lineage, including leukemia, acute lymphocitic leukemia, acute lymphoblastic leukemia, B-cell lymphoma, T-cell lymphoma, Hodgkin's lymphoma, non-Hodgkin's lymphoma, hairy cell lymphoma and Burkitt's lymphoma; hematopoietic tumors of myeloid lineage, including acute and chronic myelogenous leukemia, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannoma; and other tumors, including melanoma, seminoma, teratocarcinoma, osteosarcoma, xeroderma pigmentosum, keratoxanthoma, thyroid follicular cancer, Kaposi's sarcoma and mesothelioma.

Furthermore, a derivative of formula (Ia) as defined above is for use in a method of treating specific cellular proliferation disorders such as, for example, benign prostate hyperplasia, familial adenomatosis polyposis (FAP), neurofibromatosis, psoriasis, vascular smooth cell proliferation associated with atherosclerosis, pulmonary fibrosis, arthritis, glomerulonephritis and post-surgical stenosis and restenosis.

In addition, a derivative of formula (Ia) as defined above is for use in a method of inhibiting tumor angiogenesis and metastasis, as well as in a method of treating organ transplant rejection and host versus graft disease.

The present invention also provides a method for treating cancer, which comprises administering to a mammal in need thereof an effective amount of a derivative of formula (Ia) as defined above. The mammal in need thereof may be for example a human.

Moreover the invention provides the use of a derivative of formula (Ia) or a pharmaceutically acceptable salt thereof, as defined above, in the manufacture of a medicament for treating cancer, cellular proliferation disorders and viral infections.

Finally, the invention provides the use of anthracycline drug reagents (I), as defined above, in the preparation of conjugates.

The anthracycline drug reagents of formula (I), as defined above, can be prepared according to the general synthetic process described hereafter in Scheme A.

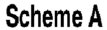

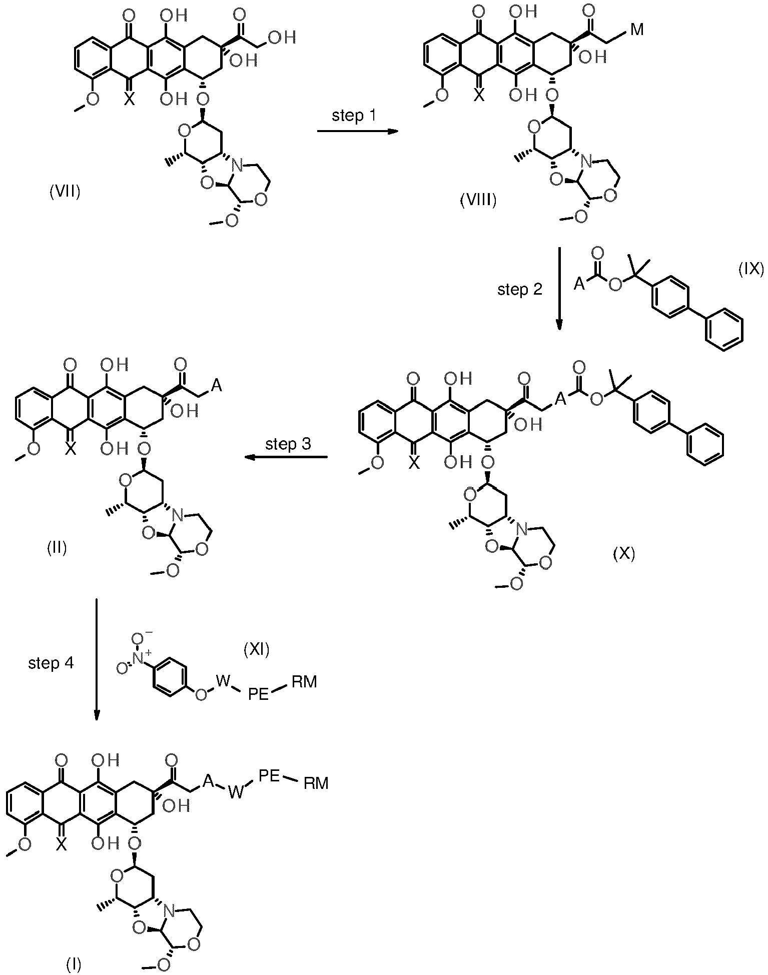

Accordingly, the process foresees the following steps:

### Step 1) reaction of a compound of formula (VII):

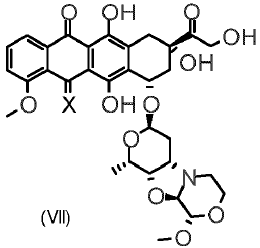

wherein **X** is a O or NH, with a suitable regent in order to introduce a leaving group **M** selected from: mesilate, tosilate and halogen, to obtain a compound of formula (VIII)

### Step 2) reaction of the so obtained compound of formula (VIII):

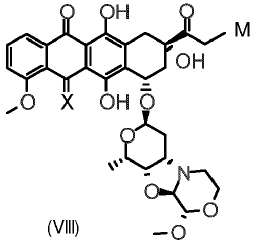

wherein **X** and **M** are as defined above in Step 1, with a suitable heterocyclic or carbocyclic intermediate of formula (IX): 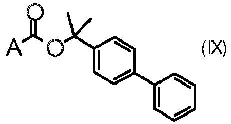 wherein **A** is a heterocycle or carbocycle selected from the group consisting of (IIIa)-(IIIe), wherein: **R** is independently hydrogen, a substituted straight or branched (C₁-C₆) alkyl and **n** is an integer from 0 to 6.

### Step 3) deprotection of resultant compound of formula (X):

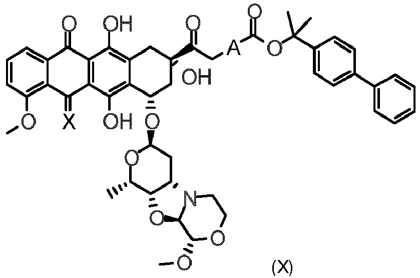

wherein **X** and **A** are as defined above in Step 1 and Step 2, by reaction with the suitable acid, to give a compound of formula (II).

### Step 4) reaction of the resultant compund (II):

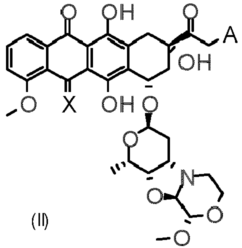

wherein **X** and **A** are as defined above in Step 1 and Step 2, with the suitable activated compund of formula (XI): 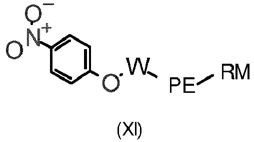 wherein **W** is independently null or a group selected from the formulas (Va)-(Vc), **PE** is null or a dipeptidic or tripeptidic moiety as define above and **RM** is an optionally reactive moiety selected from the formulas (Vla)-(Vlc), to give a compound (I).

According to **step 1)** the reaction of a compound of formula (VII), to introduce the leaving group, can be performed in a variety of ways and experimental conditions which are widely known in the art. In particular the reaction can carried out with methanesulfonic anhydride, in an organic solvent, preferably DCM, at room temperature or below, in the presence of a suitable base, preferably DMAP, collidine or pyridine, for a time varying from about 30 minutes to about 24 hours.

According to **step 2)** the reaction of a compound of formula (VIII) with a compound of formula (IX) can be accomplished in a variety of ways. This reaction may be carried out in a suitable organic solvent, preferably DCM, acetone or THF, at a temperature ranging from room temperature to 40 C° and for a time varying from about 30 minutes to about 24 hours.

According to **step 3)** the removal of the carbamate protecting group on a compound of formula (X) can be accomplished in a variety of ways and experimental conditions, which are widely known in the art (see e.g. Protective Groups in Organic Synthesis; Theodora W. Greeen, Peter G. M. Wuts 4th edition). The following acid conditions may be employed: hydrogen chloride, acetic acid, trifluoroacetic acid, trichloroacetic acid or dichloroacetic acid in solvents such as tetrahydrofuran or dichloromethane, at room temperature or below.

According to the **step 4)** the reaction of a compound of formula (II) with a compound of formula (XI) can be accomplished in a variety of ways and experimental conditions, which are widely known in the art for the preparation of carbamate. The reaction can be performed in a suitable organic solvent, preferably DMF, in the presence of a suitable base, preferably TEA or DIPEA, at a temperature ranging from room temperature to 100 C° and for a time varying from about 1 hour to about 24 hours.

The compound of general formula (VII), wherein **X** is an O atom, is commercially available or can be prepared with known methods.

The compound of general formula (VII), wherein **X** in NH, can be prepared as described in literature (D. Holte et al., Bioorganic & Medicinal Chemistry Letters 30 (2020), 127640).

The compound of general formula (IX), wherein **A** is a heterocycle or carbocycle selected from the group consisting of (IIIa)-(IIIe), can be prepared as describe below in the experimental part or starting from commercially available intermediate through procedure and experimental conditions which are widely known in the art.

The compounds of the formula (XI) are either commercially available or can be prepared with known methods.

From all of the above it is clear to the skilled person that any compound of formula (I) bearing a functional group which can be further derivatized to another functional group, by working according to methods well known in the art thus leading to other compounds of the formula (I), is intended to be comprised within the scope of the present invention. When preparing the compounds of general formula (I) according to any of the above variants of the process, optional functional groups within the starting materials, the reagents or the intermediates thereof, and which could give rise to unwanted side reactions, need to be properly protected according to conventional techniques (see e.g., Green, Theodora W. and Wuts, Peter G.M. - Protective Groups in Organic Synthesis, Third Edition, John Wiley & Sons Inc., New York (NY), 1999). Likewise, the conversion of these latter into the free deprotected compounds may be carried out according to known procedures.

The compounds of every general formula can be further transformed in other compounds of the same general formula according to methods well known in the literature, as reported in the experimental section.

According to any variant of the process for preparing the compounds of the formula (I), the starting materials and any other reactants are known or easily prepared according to known methods.

The final compounds may be isolated and purified using conventional procedures, for example chromatography and/or crystallization and salt formation.

The ADC of formula (Ia) may be prepared by several routes, employing organic chemistry reactions, conditions, and reagents known to those skilled in the art, including: (1) reaction of a nucleophilic group or an electrophilic group of an antibody with a bivalent linker reagent, to form antibody-linker intermediate Ab-L, via a covalent bond, followed by reaction with an activated drug moiety reagent; and (2) reaction of a nucleophilic group or an electrophilic group of a drug moiety reagent with a linker reagent, to form drug-linker reagent Ant-L, via a covalent bond, followed by reaction with the nucleophilic group or an electrophilic group of an antibody. Conjugation methods (1) and (2) may be employed with a variety of antibodies, drug moieties, and linkers to prepare the antibody-drug conjugates of formula (Ia). Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Each cysteine disulfide bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

Antibody-drug conjugates may also be produced by modification of the antibody to introduce electrophilic moieties, which can react with nucleophilic substituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either galactose oxidase or sodium meta-periodate may yield carbonyl (aldehyde and ketone) groups in the protein that can react with appropriate groups on the drug (Hermanson, G.T. (1996) Bioconjugate Techniques; Academic Press: New York, p 234-242). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Reactive nucleophilic groups may be introduced on the anthracycline derivative compounds by standard functional group interconversions. For example, hydroxyl groups may be converted to thiol groups by Mitsunobu-type reactions, to form thiol-modified drug compounds.

Antibody drug conjugates of formula (Ia), as defined above, can be prepared according to the general synthetic process described hereafter in experimental part.

The compounds of general formula (Ia) as defined above can be converted into pharmaceutically acceptable salts. Pharmaceutically acceptable salts of the compounds of formula (Ia) also include the salts with inorganic or organic bases, e.g., alkali or alkaline-earth metals, especially sodium, potassium, calcium, ammonium or magnesium hydroxides, carbonates or bicarbonates, acyclic or cyclic amines.

Pharmaceutically acceptable salts of the compounds of formula (Ia) include the salts with inorganic or organic acids, e.g., nitric, hydrochloric, hydrobromic, sulfuric, perchloric, phosphoric, acetic, trifluoroacetic, propionic, glycolic, fumaric, lactic, oxalic, malonic, malic, maleic, tartaric, citric, benzoic, cinnamic, mandelic, methanesulfonic, isethionic and salicylic acid.

The compounds of formula (Ia) as defined above, or the pharmaceutically acceptable salts thereof, can be subsequently formulated with a pharmaceutically acceptable carrier or diluent to provide a pharmaceutical composition. The synthesis of a compound of general formula (I), according to the synthetic processes described above, can be conducted in a stepwise manner, whereby each intermediate is isolated and purified if needed by standard purification techniques, like, for example, column chromatography, before carrying out the subsequent reaction. Alternatively, two or more steps of the synthetic sequence can be carried out in a so-called "one-pot" procedure, as known in the art, whereby only the compound resultant from the two or more steps is isolated and purified.

If a stereogenic center or another form of an isomeric center is present in a compound of the present invention, all forms of such isomer or isomers, including enantiomers and diastereomers, are intended to be covered herein. Compounds containing a stereogenic center may be used as a racemic mixture, an enantiomerically enriched mixture, or the racemic mixture may be separated using well-known techniques and an individual enantiomer may be used alone. Such separation procedures comprise standard chromatographic techniques, including chromatography using a chiral stationary phase, or crystallization. General methods for separation of compounds containing one or more asymmetric centers are reported, for instance, in Jacques, Jean; Collet, André; Wilen, Samuel H., Enantiomers, Racemates, and Resolutions, John Wiley & Sons Inc., New York (NY), 1981.

In cases in which compounds have unsaturated carbon-carbon double bonds, both the cis (Z) and trans (E) isomers are within the scope of this invention.

In cases when compounds can exist in tautomeric forms, each form is contemplated as being included within this invention whether existing in equilibrium or predominantly in one form.

### EXPERIMENTAL PART

The short forms and abbreviations used herein have the following meaning:

| | |
|---|---|
| g (gram) | mg (milligram) |
| mL (millilitre) | µL (microliter) |
| mM (millimolar) | mmol (millimole) |
| µM (micromolar) | MHz (Mega-Hertz) |
| h hour(s) | Hz (Hertz) |
| mm (millimetres) | ppm (parts per million) |
| cm (centimetres) | min (minutes) |
| µm (micron) | M (molar) |
| nM (nanomolar) | TRZ (trastuzumab) |
| FBS (fetal bovine serum) | HRMS (high-resolution mass spectra) |
| BSA (bovine serum albumin) | DTT (dithiothreitol) |
| NADPH (Nicotinamide adenine dinucleotide phosphate) | Rt (retention time) |
| 2-HG (2-Hydroxy glutaric acid) | KOtBu (potassium *tert*-butoxide) |
| rt (room temperature) | TEA (triethylamine) |
| DMAP (4-dimethylaminopyridine) | DCA (2,2 dichloroacetic acid) |
| TFA (trifluoroacetic acid) | Na₂SO₄ (sodium sulphate) |
| Et₂O (Diethyl ether) | NaHCO₃ (sodium bicarbonate) |
| AcOH (acetic acid) | ESI (electrospray ionization) |
| Na₂CO₃ (sodium carbonate) | K₂CO₃ (potassium carbonate) |
| CS₂CO₃ (caesium carbonate) | K₃PO₄ (potassium phosphate) |
| LiOH (lithium hydroxide) | NaOH (sodium hydroxide) |
| KOH (potassium hydroxide) | p-TsOH (*p*-toluensulfonic acid) |
| EtOAc (ethyl acetate) | LiHMDS (lithium bis(trimethylsilyl)amide) |
| NMP (N-methyl-2-pyrrolidone) | NaH (sodium hydride) |
| DMA (N,N-dimethylacetamide) | KH (potassium hydride) |
| DMF (N,N-dimethylformamide) | DCM (dichloromethane) |
| DIPEA (N,N-diisopropyl-N-ethylamine) | hex (hexane) |
| THF (tetrahydrofuran) | DMSO (dimethylsulfoxide) |
| MeOH (methanol) | ACN (acetonitrile) |
| EtOH (ethanol) | Bn (benzyl) |
| -OMs (mesylate) | -OTs (tosylate) |
| HOBT (N-hydroxy-benzotriazole) | DCC (1,3-dicyclohexylcarbodiimide) |
| NMR (Nuclear magnetic resonance) | MS (mass spectroscopy) |
| m/z (mass to charge ratio) | LC (Liquid chromatography) |
| MgCl₂ (Magnesium chloride) | TLC (thin layer chromatography) |
| ADC (antibody drug conjugate) | TCEP (tris(2-carboxyethyl)phosphine) |
| DAR (Drug to Antibody Ratio) | PBS (phosphate buffered saline) |
| SDS-PAGE (sodium dodecyl sulphate-polyacrylamide gel electrophoresis) | |
| PLRP LC/MS (Polymeric Reversed Phase Liquid Chromatography/Mass Spectrometry) | |
| HPLC-MS (high performance liquid chromatography - mass spectroscopy) | |
| EDCI (1-ethyl-3-(3-dimethylaminopropyl)carbodiimide hydrochloride) | |
| TBTU (N,N,N',N'-tetramethyl-O-(benzotriazol-1-yl)uronium-tetrafluoroborate) | |
| RP-HPLC (reverse phase high performance liquid chromatography) | |

### PHARMACOLOGY

The preparation and evaluation of the cytotoxicity of the ADCs of formula (Ia) is assessed as described below.

### ADCs preparation (general procedure)

The ADCs preparation has been performed by conjugatingthe antibody Trastuzumab TRZ with compounds of formula (I) through partial reduction of interchain disulfide bridges and subsequent derivatization of the free cysteine residues. In particular, TRZ was treated using a range between 1.9 to 2.3 molar equivalents of reducing agent tris(2-carboxyethyl)phosphine (TCEP) at temperature ranging between 0 to 20°C from 2 to 20 hours in a polar organic solvent, preferaby DMA, CH3CN and/or DMSO

The excess of compound (I) was quenched with cysteine 1 mM final and the resulting conjugate (Ia) was purified using a desalting column.

All ADCs of formula (Ia) have been characterized by SDS-PAGE analysis under reducing and not reducing conditions, Size Exclusion Chromatography (SEC), PLRP LC/MS and Hydrophobic Interaction Chromatography (HIC).

Average DAR has been calculated by UV absorbance at different wavelengths obtained by PLRP LC analysis.

### Proliferation assay

Cells were seeded in 96-well plates at a final density of 2000 - 4000 cells/well in appropriate medium plus 10% FBS. After 24 - 48 hours, cells were treated with serial compound (Ia) dilutions in duplicate. Cell viability was measured 144 hours later using the Cell Titer Glo assay (Promega). IC₅₀s were calculated using sigmoidal fitting software (Assay Explorer MDL). Experiments were repeated at least 2 times (n ≥ 2).

In particular, the cytotoxicity of ADCs depicted below has been assessed **(Table 1).**

### In vivo efficacy experiments

The therapeutic effect of the ADC compounds, were assessed in animal models of human transplanted tumors.

Athymic CD-1 nu/nu female mice (5 or 7 weeks old) were purchased from Charles River Laboratories (Calco, Italy) and maintained at 22-24°C under pathogen-limiting conditions as required. Cages, bedding, and food were autoclaved before use. Mice were provided with a standard diet and water ad libitum and acclimatized for 2 weeks before the start of the experiments. Housing and all procedures involving the mice were performed according to the protocol approved by the Institutional Animal Care and Use Committee of the Italian Ministry of Health (Authorization no. 292/2017-PR). Xenografts were generated by subcutaneous injection into the right flank of mice of 106 HCC-1954 HER2+ human breast cancer cells (ATCC) in 200 µl of PBS. When xenografts became palpable (approximately 150 mm³), animals were divided into groups (8 mice/group) to provide a similar range of tumor sizes for each group. At day 8 after cell inoculation, the treated groups received intravenous injections of Trastuzumab or ADCs in PBS at the indicated doses (single administration), whereas the control group (vehicle) received PBS only. The tumor volume was monitored weekly by a caliper and calculated using the following formula: tumor volume (mm³) = (length x width²)/2. The mice were considered tumor free when the tumors were no longer palpable. The animals were weighed during the observation period and toxicity was evaluated based on body weight reduction.

**Table 1** below shows cytotoxic activity (IC50 nM) of ADC compounds of formula (Ia) in HCC 1954 and NCI-N87 cell lines while, **Figure 1** and **Table 2** show *in vivo* activity data on HCC-1954 xenograft model of ADC compound (Ia^{iv}) of the present invention.

**Table 1**

| Cell line | ADC (Ia^{III}) IC₅₀ (nM) | ADC IC₅₀ (Ia^{IV}) (nM) | ADC (Ia^{X}) IC₅₀ (nM) |
|---|---|---|---|
| HCC1954 (HER++) | 0.106 | 0.188 | 0.02 |
| NCI-N87 (HER++) | 0.062 | 0.111 | 0.015 |

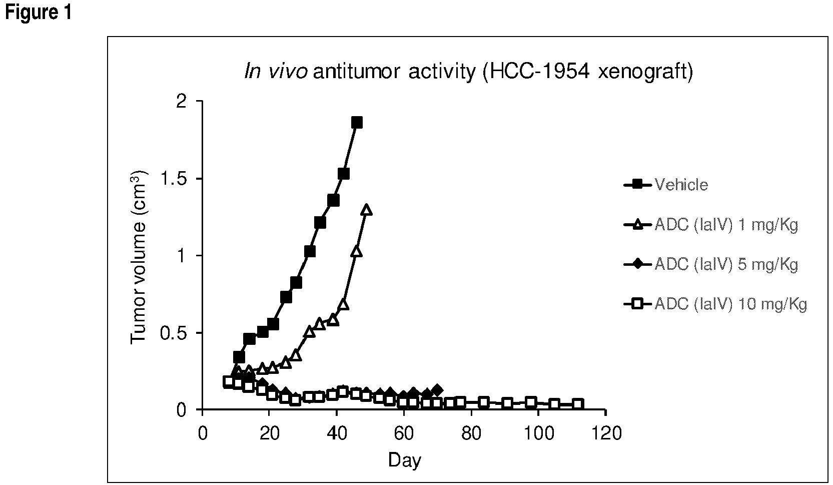

**Table 2**

| Compound | Dose mg/Kg | Tox | Tumor free animals at day 113 |
|---|---|---|---|
| ADC (Ia^{IV}) | 1 | 0/8 | 0/8 |
| ADC (Ia^{IV}) | 5 | 0/8 | 5/8 |
| ADC (Ia^{IV}) | 10 | 0/8 | 8/8 |

ADC (Ia^{IV}) was tested in a xenograft model of HCC-1954 (human breast cancer cell line, ErbB2+) inoculated subcutaneously in athymic nude mice. Both ADCs were administered intravenously (single treatment) eight days after tumor injection and randomization (8 animals/group, average tumor volume: 0.18 cm3). ADC (IaI^{V}) was administered at 1, 5 or 10 mg/Kg showing dose-dependent tumor growth inhibition without evident toxic effects at any dose: at the end of the experiment (113 days) 0/8 animals were tumor free at 1 mg/Kg, whereas 5/8 and 8/8 mice were found tumor free at 5 mg/Kg and 10 mg/Kg, respectively.

These data show that ADC (Ia^{IV}) has a remarkable therapeutic index, showing tumor eradication in 8/8 animals and good tolerability at 10 mg/kg, thus indicating that the ADC of the present invention are suitable to be used in the treatment of cancer.

### PREPARATION OF COMPOUNDS OF FORMULA (I)

For a reference to any specific compound of formula (I) of the invention, optionally in the form of a pharmaceutically acceptable salts, see the experimental section and claims. Referring to the examples that follow, compounds of the present invention were synthesized using the methods described herein, or other methods, which are well known in the art. With the aim at better illustrating the present invention, without posing any limitation to it, the following examples are given. As used herein the symbols and conventions used in the processes, schemes and examples are consistent with those used in the contemporary scientific literature, for example, the Journal of the American Chemical Society or the Journal of Biological Chemistry*.*

Compound names are IUPAC names, generated by using Biovia Draw 2020 (by Dassault Systemes).

Unless otherwise noted, all materials, including anhydrous solvent such as DMF, THF, DCM, were obtained from commercial suppliers, of the best grade and used without further purification. All reactions involving air- or moisture-sensitive compounds were performed under nitrogen or argon atmosphere.

### General purification and analytical methods

Flash Chromatography was performed on silica gel (Merck grade 9395, 60A).

### HPLC LCQ method

HPLC-MS/UV analyses were performed on a LCQ DecaXP (Thermo, San Jose, US) ion trap instrument, equipped with an electrospray (ESI) ion source. The mass spectrometer is connected to a Surveyor HPLC system (Thermo, San Jose, US) with an UV photodiode array detector (UV detection 215-400 nm). A Waters XSelect CSH C18 column 50x4.6 mm, 3.5 µm particle size was used. Mobile phase A was ammonium acetate 5 mM buffer (pH 4.5 with acetic acid):acetonitrile 95:5, and mobile phase B was ammonium acetate 5 mM buffer (pH 4.5 with acetic acid): acetonitrile 5:95. Gradient from 0 to 100 % B in 7 minutes, hold 100% B 2 minutes. Flow rate 1 mL/min. Injection volume 10 µL. Retention times (HPLC r.t.) are given in minutes. Full scan, mass range from 50 to 1200 amu. Heated capillary temp was 200°C and Spray voltage value was set at 4kV. Mass are given as m/z ratio.

Instrument control, data acquisition and processing were performed by using Xcalibur 1.4 SR1 software (Thermo).

### HPLC LCT Method

HPLC-MS/UV analyses and High Resolution Mass Spectra (HRMS) were performed on a A Waters Alliance LC 2795 equipped with a Waters PDA UV detector 2996 and a TOF Waters LCT Premier XE mass detector (ESI interface) supported by a Waters Reagent Manager liquid pump. The assay is based on generic gradient reversed-phase chromatography that allows complementing an identity-purity assay with determination and confirmation of the expected exact mass of the compounds in the same run. Compound identity is accomplished by on-line serial ESI(+) Full Scan MS detection, sample purity is obtained as relative "Area Percent" of the integrated LC/UV trace at 216-400 nm. The liquid chromatograph is equipped with a Waters XBridge CSH C18 column (3.0×30 mm, 3.5 µm particle size) thermostated at 50 °C. Alternatively a Supelco column Ascentis Express C18 (2.7x 30mm × 3um) was used.

Mobile phases A was 0.05% w/v formic acid in highly purified water and mobile phase B was a 70/25/5 (v/v/v) mixture of MeOH/iPrOH/H2O containing 0.035% w/v of formic acid. Gradient from 0 to 100 % B in 17.5 minutes, hold 100% B 5 minutes. Flow rate 0.8 mL/min, Injection volume 4 µL. The ESI source operated at 100 °C, 2.5 kV capillary voltage, 60 V cone, 600 L/hr nitrogen desolvation flow at 350 °C and 10 L/hr nitrogen cone flow. The "Normal" Zfocus is set at 140. The analyzer is normally optimized at 7200 V flight tube.

In order to obtain high resolution mass spectra, the eluent from the HPLC column was split and 25 µL/min were mixed with a 100 µL/min stream of a 30/10/60 (v/v/v) mixture of MeOH/iPrOH/H2O containing 0.01% w/v of formic acid and 80 nM Trimethoprim coming from a Waters Reagent Manager pump before entering the MS source. Trimethoprim was chosen as stable, soluble and appropriate reference compound for real-time single-point mass correction. ES(+) full scan 80-1200 amu centroided data acquisition was carried out at 2 Hz sampling rate in the "W" mode. The LCT embedded PC provided both real-time data centroiding and real-time mass correction based on the Trimethoprim. H+ reference mass of 291.1452 Da. Proper intensity MS spectra (40 to 2000 analyte counts) were averaged to obtain the final result.

The preparative HPLC equipment consisted of a Shimadzu HPLC system equipped with SCL-8A System Controller, two LC-8A Pumps, SPD-6A UV Spectrophotometric Detector and manual Rheodyne injection system. Data acquisition (analogic signal) and data processing were provided by Empower 2 software. Purification was carried out at 25 °C at a flow rate of 15mL/min using a Waters X-Terra MS RP18 (150 × 30 mm, 10 µm) column. Mobile phase A was 0.1% TFA in water/acetonitrile (95:5) or, alternatively, Mobile phase A was 0.05% NH3 in water/acetonitrile (95:5) and mobile phase B was H₂O/acetonitrile (5:95); the gradient was from 10 to 90% B in 15 minutes then ramp to 100% B in 0.1 minutes. Injection volume max 500 µL.

¹H-NMR spectra were recorded at a constant temperature of 28 °C on a Varian INOVA 400 spectrometer operating at 400.5 MHz and equipped with a 5 mm ¹H{¹⁵N-³¹P} z-axis PFG Indirect Detection probe and on a Varian INOVA 500 spectrometer operating at 499.7 MHz and equipped with a 5 mm ¹H{¹³C-¹⁵N} triple resonance Indirect Detection probe. Chemical shifts were referenced with non deuterated residual solvent signal (DMSO-*d₅*: 2.50 ppm for ¹H). Data are reported as follows: chemical shift (δ), multiplicity (s = singlet, d = doublet, t = triplet, q = quartet, qt = quintet, br. s = broad singlet, dd = doublet of doublets, ddd = doublet of doublets of doublets, m = multiplet), coupling constants (J, Hz) and number of protons.

The synthetic preparation of some compounds of formula (I) of the invention is described in the following examples. The compounds of the present invention, as prepared according to the following examples, were also characterized by ¹H NMR and/or by HPLC/MS analytical data; HPLC/MS data were collected following any one of methods LCQ or LCT.

### Example A

### Intermediates IX

### [1-methyl-1-(4-phenylphenyl)ethyl] piperazine-1-carboxylate

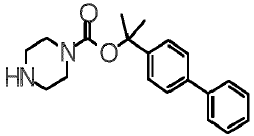

To a solution of piperazine (0.517 g, 6 mmol, 3 eq.) in DMF (2.5 mL), methyl 4-[1-methyl-1-(4-phenylphenyl) ethoxy] carbonyloxybenzoate (0.781 g, 2 mmol, 1 eq.) was added. Reaction was stirred overnight at room temperature. HPLC-MS analysis shown the presence of the title compound. EtOAc was added to the mixture and organic layer was washed with water three times, dried over Na₂SO₄, filtrated and evaporated to dryness. The residue was purified by chromatographic column DCM/MeOH (100/5 to 100/9) to give the title compound as a solid (0.600 g, 1.85 mmol, 92% yield).

¹H NMR (500 MHz, DMSO-*d6*) δ ppm 1.72 (s, 6 H) 2.57 - 2.75 (m, 5 H) 3.17 (d, *J*=5.34 Hz, 2 H) 3.37 - 3.51 (m, 2 H) 7.34 - 7.37 (m, 1 H) 7.40 (d, *J*=8.39 Hz, 2 H) 7.46 (t, *J*=7.70 Hz, 2 H) 7.60 - 7.68 (m, 4 H)

HRMS (ESI) calcd for C₂₀H₂₄N₂O₂ [M + H]⁺ 347.1730, found 347.1735

Operating in an analogous way, but employing suitable substituted starting material the following compounds were obtained:

### [1-methy)-1-(4-phenylphenyl)ethyl]4-(methylamino)piperidine-1-carboxylate

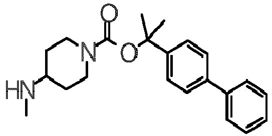

¹H NMR (500 MHz, DMSO-*d6*) δ ppm 0.99 - 1.27 (m, 2 H) 1.72 (s, 6 H) 1.75 - 1.90 (m, 2 H) 2.24 (br. s., 1 H) 2.29 (s, 3 H) 2.69 - 3.09 (m, 2 H) 3.10 - 3.50 (m, 1 H) 3.75 (br. s., 1 H) 3.98 (br. s., 1 H) 7.30 - 7.38 (m, 1 H) 7.40 (d, J=8.54 Hz, 2 H) 7.46 (t, J=7.70 Hz, 2 H) 7.62 (d, J=8.54 Hz, 2 H) 7.64 - 7.68 (m, 2 H)
HRMS (ESI) calcd for C₂₂H₂₈N₂O₂ [M + H]⁺ 353.2224, found 353.2219

### [1-methyl-1-(4-phenylphenyl)ethyl] N-methyl-N-(4-piperidyl)carbamate

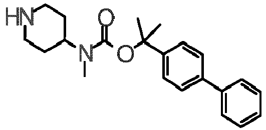

¹H NMR (600 MHz, DMSO-*d6*) δ ppm 1.34 -1.65 (m, 4H) 1.73 (s, 6 H) 2.41 (m, 1H) 2.59-3.09 (m, 6H) 3.72- 3.96 (m, 2H) 7.33 - 7.37 (m, 1 H) 7.38 - 7.42 (m, 2 H) 7.43 - 7.48 (m, 2 H) 7.61 (d, J=8.36 Hz, 2 H) 7.65 (dd, J=8.27, 1.00 Hz, 2 H)
HRMS (ESI) calcd for C₂₂H₂₈N₂O₂ [M + H]⁺ 353.2224, found 353.2227

### [1-methyl-1-(4-phenylphenyl)ethyl] 1,4-diazepane-1-carboxylate

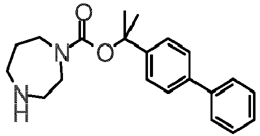

¹H NMR (600 MHz, DMSO-*d6*) δ ppm 1.58 (t, J=5.63 Hz, 1 H) 1.73 (s, 6 H) 1.74 - 1.79 (m, 1 H) 2.65 - 2.71 (m, 2 H) 2.75 (t, J=5.81 Hz, 1 H) 2.81 - 2.87 (m, 1 H) 3.20 - 3.25 (m, 1 H) 3.29 - 3.40 (m, 2 H) 3.44 - 3.48 (m, 1 H) 3.52 (t, J=6.09 Hz, 1 H) 7.32 - 7.38 (m, 1 H) 7.39 - 7.44 (m, 2 H) 7.44 - 7.48 (m, 2 H) 7.61 (d, J=7.99 Hz, 2 H) 7.63 - 8.51 (m, 2 H)
HRMS (ESI) calcd for C₂₁H₂₆N₂O₂ [M + H]⁺ 339.2067, found 339.2071

### Step 1

### [2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricylo[7.4.0.02,7]tridecan-4-yl]oxy]-11-oxo-3,4-dihydro-1H-tetracen-2-yl]ethyl] methanesulfonate (VIII)

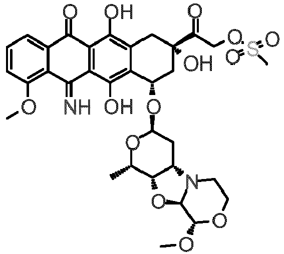

To a solution of (8S,10S)-6,8,11-trihydroxy-8-(2-hydroxyacetyl)-12-imino-1-methoxy-10-[[(2S,4R,6S,7S,9R,10S)-10-methoxy-6-methyl-5,8,11-trioxa-1-azatricyclo[7.4.0.02,7]tridecan-4-yl]oxy]-9,10-dihydro-7H-tetracen-5-one (0.025 g, 0.039 mmol, 1 eq.), 2,4,6-collidine (0.019 g, 0.156 mmol, 4 eq.) and 4-(dimethylamino)pyridine (0.005 g, 0.039 mmol, 1 eq.) in DCM dry (5 mL), methanesulfonic anhydride (0.027 g, 0.156 mmol, 4 eq.) was added. Reaction was stirred at room temperature for 5 hours. HPLC-MS analysis shown the presence of the title compound. Water and DCM were added in the mixture, organic layer was washed with water three times, dried over Na₂SO₄, filtrated and evaporated to dryness. The crude was triturated with Et₂O and filtrated three times to afford the title compound as a blue solid (0.028 g, 0.039 mmol, 100% yield).
HRMS (ESI) calcd for C₃₃H₃₈N₂O₁₄S [M + H]⁺ 719.2117, found 719.2122

Operating in an analogous way, but employing suitable substituted starting material the following compound was obtained:

### [2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl] methanesulfonate (VIII)

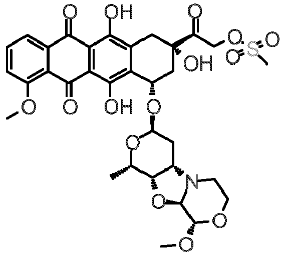

Red solid (0.0035 g, 0.0049 mmol, 74% yield).
¹H NMR (500 MHz, DMSO-*d*₆) δ ppm 1.24 (d, *J*=6.10 Hz, 3 H) 1.64 - 1.72 (m, 2 H) 2.13 - 2.19 (m, 1 H) 2.27 (d, *J*=15.50 Hz, 1 H) 2.60 - 2.73 (m, 2 H) 2.90 - 3.11 (m, 2 H) 3.26 (s, 3 H) 3.31 (s, 3 H) 3.35 - 3.41 (m, 1 H) 3.47 - 3.53 (m, 1 H) 3.62 - 3.71 (m, 1 H) 3.94 (d, *J*=6.35 Hz, 1 H) 4.00 (s, 3 H) 4.12 - 4.20 (m, 1 H) 4.21 - 4.25 (m, 1 H) 4.59 (s, 1 H) 5.02 (br. s., 1 H) 5.24 (t, *J*=4.27 Hz, 1 H) 5.37 - 5.50 (m, 2 H) 5.58 (s, 1 H) 7.59 - 7.72 (m, 1 H) 7.93 (d, *J*=4.27 Hz, 2 H) 13.28 (br.s., 1 H) 14.07 (br. s., 1 H)
HRMS (ESI) calcd for C₃₃H₃₇NO₁₅S [M + H]⁺ 720.1957, found 720.1969

### Step 2

### [1-methyl-1-(4-phenylphenyl)ethyl]4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate (X) (X = NH, A = (IIIb') wherein R is a methyl group)

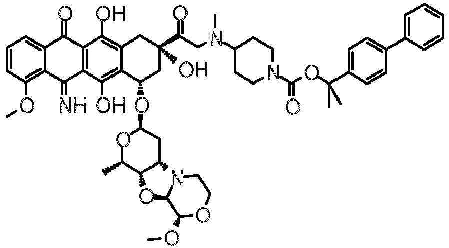

[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl] methanesulfonate (0.028 g, 0.039 mmol, 1 eq.) and [1-methyl-1-(4-phenylphenyl)ethyl] 4-(methylamino)piperidine-1-carboxylate (0.083 g, 0.234 mmol, 6 eq.) were dissolved in DCM dry (10 mL). Solvent has been removed under reduced pressure (rotavapor) and re-dissolved in DCM dry (3 mL). Solvent has been removed under reduced pressure (rotavapor) and the sequence of solubilization and evaporation has been repeated until the stating material ([2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl] methanesulfonate) was no longer detectable by HPLC-MS and/or TLC. The crude was purified by chromatographic column DCM/Acetone (8/2 to 7/3) to give the title compound as a blue solid (0.015 g, 0.0154 mmol, 39% yield).
¹H NMR (600 MHz, DMSO-*d*₆) δ ppm 1.19 - 1.27 (m, 5 H) 1.60 - 1.64 (m, 2 H) 1.72 (m, 8 H) 2.05 - 2.11 (m, 1 H) 2.12 - 2.17 (m, 1 H) 2.23 (s, 3 H) 2.56 - 2.60 (m, 1 H) 2.63 - 2.68 (m, 2 H) 2.56 - 2.98 (br..s , 2H) 2.83 - 2.88 (m, 1 H) 2.93 - 2.98 (m, 1 H) 3.29 (s, 3 H) 3.34 (m, 1H) 3.45 - 3.51 (m, 1 H) 3.60 -3.94 (br. S., 2H) 3.62 - 3.68 (m, 1 H) 3.73 - 3.83 (m, 2 H) 3.91 - 3.93 (m, 1 H) 4.12 (s, 3 H) 4.16 (m, *J*=6.63 Hz, 1 H) 4.23 (d, *J*=2.00 Hz, 1 H) 4.58 (d, *J*=1.91 Hz, 1 H) 5.03 (t, *J*=4.29 Hz, 1 H) 5.37 (s, 1 H) 5.40 (t, *J*=4.59 Hz, 1 H) 7.31 - 7.36 (m, 1 H) 7.38 - 7.42 (m, 2 H) 7.44 (t, *J*=7.74 Hz, 2 H) 7.62 (d, *J*=8.49 Hz, 2 H) 7.63 - 7.67 (m, 3 H) 7.87 (t, *J*=8.08 Hz, 1 H) 8.08 (dd, *J*=7.83, 0.98 Hz, 1 H) 9.67 (d, *J*=4.68 Hz, 1 H) 13.60 (d, *J*=5.04 Hz, 1 H) 15.89 (br. s., 1 H)
HRMS (ESI) calcd for C₅₄H₆₂N₄O₁₃ [M + H]⁺ 975.4386, found 975.4381

Operating in an analogous way, but employing suitable substituted starting materials (intermediates VIII and IX) the following compounds were obtained:

### [1-methyl-1-(4-phenylphenyl)ethyl] 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]piperazine-1-carboxylate (X) (X = NH, A = (IIIa'))

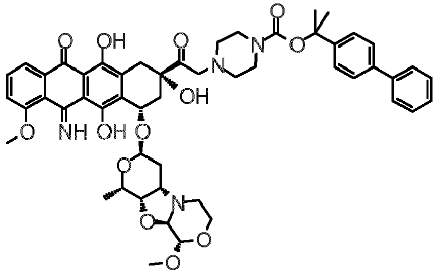

Blue solid (0.016 g, 0.017 mmol, 60% yield).
¹H NMR (500 MHz, DMSO-*d6*) δ ppm 1.21- 1.28 (m, 3 H) 1.59 - 1.66 (m, 2 H) 1.72 (s, 6 H) 2.05 - 2.18 (m, 2 H) 2.36 - 2.49 (m, 4 H) 2.58 - 2.71 (m, 2 H) 2.82 - 2.97 (m, 2 H) 3.18 - 3.38 (m, 5H) 3.31 (s, 3H) 3.47 - 3.55 (m, 2 H) 3.62 - 3.75 (m, 3 H) 3.94 (d, J=7.52 Hz, 1 H) 4.12 (s, 3 H) 4.14 - 4.18 (m, 1 H) 4.24 (d, J=1.82 Hz, 1 H) 4.60 (d, J=1.69 Hz, 1 H) 5.01 - 5.09 (m, 1 H) 5.38 (s, 1 H) 5.41 (t, J=4.41 Hz, 1 H) 7.33 - 7.38 (m, 1 H) 7.40 (d, J=8.43 Hz, 2 H) 7.47 (t, J=7.65 Hz, 2 H) 7.62 (d, J=8.30 Hz, 2 H) 7.66 (d, J=7.65 Hz, 2 H) 7.86 (t, J=7.65 Hz, 1 H) 8.09 (d, J=7.78 Hz, 1 H) 9.67 (br. s., 1 H) 13.59 (br. s., 1 H) 15.90 (br. s., 1 H)
HRMS (ESI) calcd for C₅₂H₅₈N₄O₁₃ [M + H]⁺ 947.4073, found 947.4069

### [1-methyl-1-(4-phenylphenyl)ethyl] N-methyl-N-[1-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-4-piperidyl]carbamate (X) (X = NH, A = (IIIc') wherein R is a methyl group)

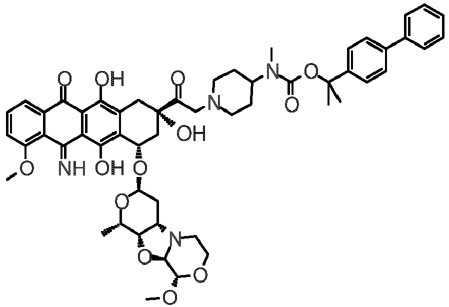

Blue solid (0.008 g, 0.0082 mmol, 49% yield).
¹H NMR (600 MHz, DMSO-*d6*) δ ppm 1.15- 1.42 (m, 7 H) 1.62 -(m, 2 H) 1.72 (s, 6 H) 1.95-2.19 (m, 4 H) 2.52- 3.00 (m, 9 H) 3.30 (s, 3 H) 3.34 - 3.39 (m, 1 H) 3.37 (m, 1 H) 3.47 - 3.75 (m, 4 H) 3.66 (t, J=9.20 Hz, 1 H) 3.91 - 3.95 (m, 1 H) 4.07 - 4.18 (m, 4 H) 4.23 (d, J=1.54 Hz, 1 H) 4.59 (d, J=1.91 Hz, 1 H) 5.05 (br. s., 1 H) 5.40 (br. s., 2 H) 7.31 - 7.43 (m, 3 H) 7.46 (t, J=7.74 Hz, 2 H) 7.54 - 7.68 (m, 5 H) 7.87 (t, J=8.08 Hz, 1 H) 8.04 - 8.11 (m, 1 H) 9.65 (d, J=4.59 Hz, 1 H) 13.56 (d, J=4.54 Hz, 1 H) 15.91 (br. s., 1 H)
HRMS (ESI) calcd for C₅₄H₆₂N₄O₁₃ [M + H]⁺ 975.4386, found 975.4390

### [1-methyl-1-(4-phenylphenyl)ethyl] 4-[methyl-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]amino]piperidine-1-carboxylate (X) (X = O, A = (IIIb') wherein R is a methyl group)

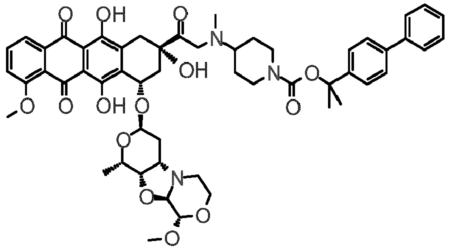

Red solid (0.0065 g, 0.0067 mmol, 51% yield)
HRMS (ESI) calcd for C₅₄H₆₁N₃O₁₄ [M + H]⁺ 976.4226, found 976.4229

### [1-methyl-1-(4-phenylphenyl)ethyl] 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]piperazine-1-carboxylate (X) (X = O, A = (IIIa'))

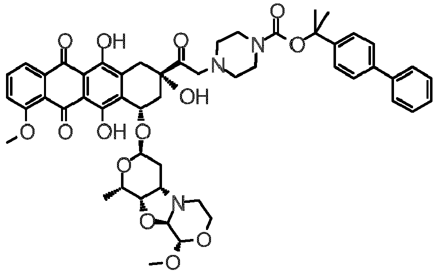

Red solid (0.052 g, 0.054 mmol, 70% yield).
HRMS (ESI) calcd for C₅₂H₅₇N₃O₁₄ [M + H]⁺ 948.3914, found 948.3904

### [1-methyl-1-(4-phenylphenyl)ethyl]N-methyl-N-[1-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl]carbamate (X) (X = O, A = (IIIc') wherein R is a methyl group)

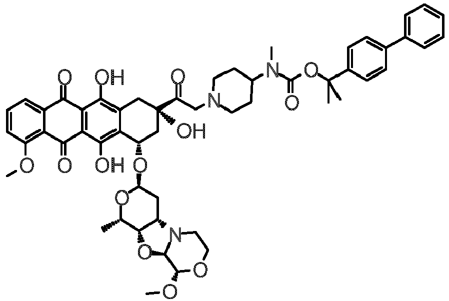

Red solid (0.0033 g, 0.0034 mmol, 61% yield)
¹H NMR (600 MHz, DMSO-*d*₆) δ ppm 1.25 (m, 3 H) 1.71 - 1.76 (m, 6 H) 1.37 -1.69 (m, 6 H) 1.96 - 2.23 (m, 4 H) 2.62 - 3.10 (m, 8 H) 3.30 (br. s., 3 H) 3.35 - 3.40 (m, 1 H) 3.47 - 3.53 (m, 1 H) 3.47 - 3.74 (m, 4 H) 3.66 (m, 1 H) 3.93 (d, *J*=5.45 Hz, 1 H) 3.99 (s, 3 H) 4.17 (d, *J*=7.13 Hz, 1 H) 4.23 (d, *J*=1.77 Hz, 1 H) 4.59 (d, *J*=1.95 Hz, 1 H) 5.00 (br. s., 1 H) 5.23 (br. s., 1 H) 5.47 (br. s., 1 H) 7.31 - 7.38 (m, 1 H) 7.39 (d, *J*=8.36 Hz, 2 H) 7.46 (t, *J*=7.74 Hz, 2 H) 7.61 (d, *J*=8.36 Hz, 2 H) 7.63 - 7.73 (m, 3 H) 7.88 - 7.96 (m, 2 H) 13.28 (br. s., 1 H) 14.06 (br. s., 1 H)
HRMS (ESI) calcd for C₅₄H₆₁N₃O₁₄ [M + H]⁺ 976.4226, found 976.4221

### [1-methyl-1-(4-phenylphenyl)ethyl] 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-1,4-diazepane-1-carboxylate (X) (X = O, A = (IIIa"))

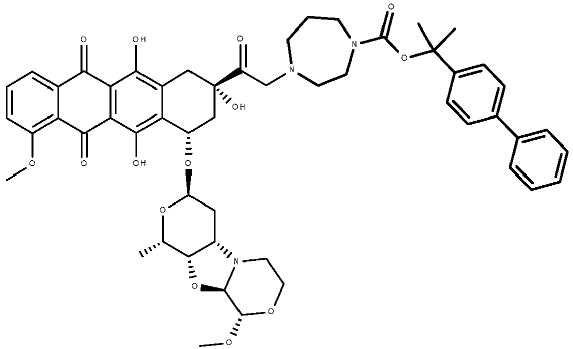

Red solid (0.0045 g, 0.0047 mmol, 64% yield)
1H NMR (600 MHz, DMSO-d6) δ ppm 1.19 - 1.28 (m, 3 H) 1.66 (m., 3 H) 1.69 - 1.75 (m, 6 H) 1.81 (m, 1 H) 2.04 - 2.26 (m, 2 H) 2.57 - 2.70 (m, 5 H) 2.79 (t, J=5.00 Hz, 1 H) 2.90 - 3.09 (m, 2 H) 3.30 (s, 3 H) 3.26 - 3.34 (m, 2 H) 3.36 (m, 1 H) 3.44 - 3.56 (m, 3 H) 3.66 (m, 1 H) 3.84 - 3.94 (m, 3 H) 3.99 (s, 3 H) 4.18 (m, 1 H) 4.23 (d, J=1.82 Hz, 1 H) 4.58 (d, J=2.00 Hz, 1 H) 4.93 - 5.06 (m, 1 H) 5.23 (m, 1 H) 5.35 - 5.51 (m, 1 H) 7.29 - 7.37 (m, 1 H) 7.38 - 7.49 (m, 4 H) 7.54 - 7.71 (m, 5 H) 7.89 - 7.95 (m, 2 H) 13.27 (br. s., 1 H) 14.03 (s, 1 H)
HRMS (ESI) calcd for C₅₃H₅₉N₃O₁₄ [M + H]⁺ 962.4070, found 962.4073

### Step 3

### (8S,10S)-6,8,11-trihydroxy-12-imino-1-methoxy-10-[[(2S,4R,6S,7S,9R,10S)-10-methoxy-6-methyl-5,8,11-trioxa-1-azatricyclo[7.4.0.02,7]tridecan-4-yl]oxy]-8-[2-[methyl(4-piperidyl)amino]acetyl]-9,10-dihydro-7H-tetracen-5-one (II) (compound 17; X = NH, A = (IIIb') wherein R is a methyl group)

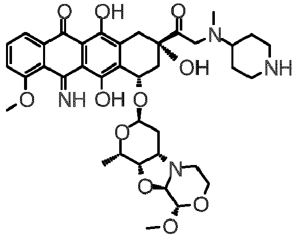

To a solution of [1-methyl-1-(4-phenylphenyl)ethyl] 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate (0.0145 g, 0.0149 mmol, 1 eq.) in DCM dry (4 mL) at T = 0°C, 2,2-dichloroacetic acid (2.0 mL, 0.297 mmol, 20 eq.) was added. Reaction was kept at T = 0° C for 15 minutes and then was stirred at room temperature for 4 hours. TLC analysis showed complete convertion of the starting material into the title product. Reaction mixture was poured into a sep funnel with a sat. acqueous solution of NaHCO₃. Aqueous layer was washed with DCM three times, the combined organic phases were dried over Na₂SO₄, filtrated and evaporated to dryness. The crude was purified by chromatographic column DCM/MeOH (95/5 to 9/1) to give the title compound as a blue solid (0.008 g, 0.01 mmol, 68% yield).
¹H NMR (600 MHz, DMSO-d*6*) δ ppm 1.23 (m, 3 H) 1.45 -1.65 (m, 4 H) 1.80 - 1.86 (m, 2 H) 1.92 - 2.0 (m, 2 H) 2.86 (m, 1 H) 2.07 (dd, *J*=14.76,6,13 1 H) 2.14 (dd, *J*=14.76, 3.22 1 H) 2.22 (s, 3 H) 2.59 - 2.69 (m, 2 H) 2.73 (m, 1) 2.82 (m, 2H) 2.92 - 2.98 (m, 1 H) 3.23 - 3.33 (m, 2 H) 3.30(s, 3 H) 3.37 (m, 1 H) 3.49 (m, 1 H) 3.66(m, 1 H) 3.79 (s, 2 H) 3.93 (dd, *J*=6.34, 1.70 Hz, 1 H) 4.10 - 4.17 (m, 4 H) 4.24 (d, *J*=2.00 Hz, 1 H) 4.59 (d, *J*=1.95 Hz, 1 H) 5.01 - 5.05 (m, 1 H) 5.36 (m, 1 H) 5.40 (t, *J*=4.61 Hz, 1 H) 7.65 (d, *J*=8.45 Hz, 1 H) 7.88 (t, *J*=8.08 Hz, 1 H) 8.07 - 8.11 (m, 1 H) 9.68 (d, J=5.00 Hz, 1 H) 13.61 (d, *J*=5.00 Hz, 1 H) 15.88 (br. s., 1 H)
HRMS (ESI) calcd for C₃₈H₄₈N₄O₁₁ [M + H]⁺ 737.3392, found 737.3389

Operating in an analogous way, but employing suitable substituted starting materials (intermediates X) the following compound was obtained:

### (8S,10S)-6,8,11-trihydroxy-12-imino-1-methoxy-10-[[(2S,4R,6S,7S,9R,10S)-10-methoxy-6-methyl-5,8,11-trioxa-1-azatricydo[7.4.0.02,7]tridecan-4-yl]oxy]-8-(2-piperazin-1-ylacety))-9,10-dihydro-7H-tetracen-5-one (II) (compound 18; X = NH, A = (IIIa'))

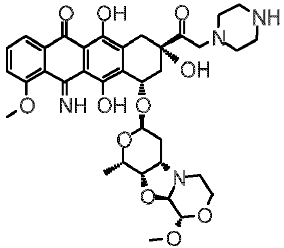

Blue solid (0.007 g, 0.0099 mmol, 72% yield).
¹H NMR (500 MHz, DMSO-*d*₆) δ ppm 1.24 (d, *J*=6.59 Hz, 3 H) 1.57 - 1.67 (m, 2 H) 2.02 - 2.09 (m, 1 H) 2.10 - 2.16 (m, 1 H) 2.29 - 2.37 (m, 4 H) 2.57 - 2.64 (m, 1 H) 2.65 - 2.72 (m, 5 H) 2.80 - 2.97 (m, 2 H) 3.31 (s, 3 H) 3.34 (m, 1 H) 3.47 - 3.53 (m, 1 H) 3.57 (d, *J*=1.10 Hz, 2 H) 3.63 - 3.70 (m, 1 H) 3.93 (d, *J*=7.87 Hz, 1 H) 4.12 (s, 3 H) 4.14 - 4.19 (m, 1 H) 4.24 (d, *J*=1.83 Hz, 1 H) 4.60 (d, *J*=1.65 Hz, 1 H) 5.03 (t, *J*=4.21 Hz, 1 H) 5.37 (s, 1 H) 5.40 (t, *J*=4.49 Hz, 1 H) 7.64 (d, *J*=8.06 Hz, 1 H) 7.87 (t, *J*=8.15 Hz, 1 H) 8.08 (d, *J*=7.87 Hz, 1 H) 9.66 (d, *J*=4.76 Hz, 1 H) 13.58 (d, *J*=4.76 Hz, 1 H) 15.65 (br. s, 1 H)
HRMS (ESI) calcd for C₃₆H₄₄N₄O₁₁ [M + H]⁺ 709.3080, found 709.3066

### (8S,10S)-6,8,11-trihydroxy-12-imino-1-methoxy-10-[[(2S,4R,6S,7S,9R,10S)-10-methoxy-6-methyl-5,8,11-trioxa-1-azatricyclo[7.4.0.02,7]tridecan-4-yl]oxy]-8-[2-[4-(methylamino)-1-piperidyl]acetyl]-9,10-dihydro-7H-tetracen-5-one (II) (compound 19; X = NH, A = (IIIc') wherein R is a methyl group)

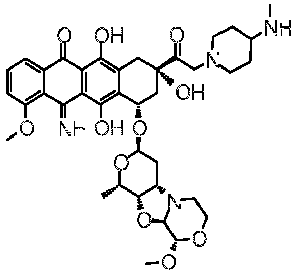

Blue solid (0.001 g, 0.0014 mmol, 19% yield)
HRMS (ESI) calcd for C₃₈H₄₈N₄O₁₁ [M + H]⁺ 737.3392, found 737.3399

### (7S,9S)-6,9,11-trihydroxy-4-methoxy-7-[[(2S,4R,6S,7S,9R,10S)-10-methoxy-6-methyl-5,8,1 1-trioxa-1-azatricyclo[7.4.0.02,7]tridecan-4-yl]oxy]-9-[2-[methyl(4-piperidyl)amino]acetyl]-8,10-dihydro-7H-tetracene-5,12-dione (II) (compound 20; X = O, A = (IIIb') wherein R is a methyl group)

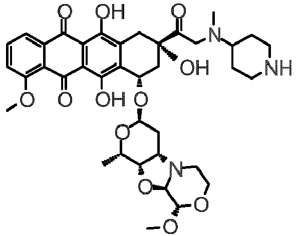

Red solid (0.0031 g, 0.0042 mmol, 38% yield)
HRMS (ESI) calcd for C₃₈H₄₇N₃O₁₂ [M + H]⁺ 738.3233, found 738.3238

### (7S,9S)-6,9,1 1 -trihydroxy-4-methoxy-7-[[(2S,4R,6S,7S,9R,10S)-10-methoxy-6-methyl-5,8,11-trioxa-1-azatricyclo[7.4.0.02,7]tridecan-4-yl]oxy]-9-(2-piperazin-1-ylacetyl)-8,10-dihydro-7H-tetracene-5,12-dione (II) (compound 21; X = O, A = (IIIa'))

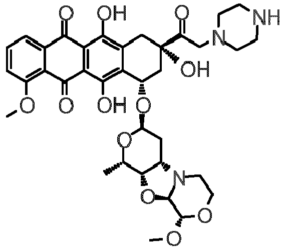

Red solid (0.011 g, 0.016 mmol, 86% yield)
¹H NMR (500 MHz, DMSO-*d*₆) δ ppm 1.24 (d, *J*=6.59 Hz, 3 H) 1.63 - 1.72 (m, 2 H) 2.08 - 2.16 (m, 1 H) 2.17 - 2.23 (m, 1 H) 2.27 - 2.39 (m, 4 H) 2.58 - 2.73 (m, 6 H) 2.87 - 3.04 (m, 2 H) 3.30(s, 3 H) 3.38(m, 1 H)3.46 - 3.54 (m, 3 H) 3.58 (s, 2 H) 3.64 - 3.70 (m, 1 H) 3.93 (d, *J*=7.08 Hz, 1 H) 3.99 (s, 3 H) 4.15 - 4.21 (m, 1 H) 4.23 (d, *J*=1.59 Hz, 1 H) 4.59 (d, *J*=1.46 Hz, 1 H) 4.99 (t, *J*=4.33 Hz, 1 H) 5.23 (t, *J*=4.33 Hz, 1 H) 5.47 (s, 1 H) 7.64 - 7.71 (m, 1 H) 7.89 - 7.95 (m, 2 H)
HRMS (ESI) calcd for C₃₆H₄₃N₃O₁₂ [M + H]⁺ 710.2920, found 710.2923

### (7S,9S)-6,9,11-trihydroxy-4-methoxy-7-[[(2S,4R,6S,7S,9R,10S)-10-methoxy-6-methyl-5,8,11-trioxa-1-azatricyclo[7.4.0.02,7]tridecan-4-yl]oxy]-9-[2-[4-(methylamino)-1-piperidyl]acetyl]-8,10-dihydro-7H-tetracene-5,12-dione (II) (compound 22; X = O, A = (IIIc') wherein R is a methyl group)

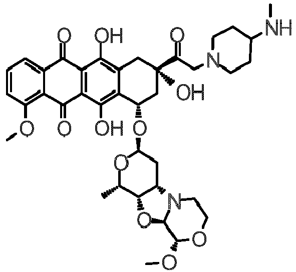

Red solid (0.007 g, 0.0095 mmol, 93% yield)
HRMS (ESI) calcd for C₃₈H₄₇N₃O₁₂ [M + H]⁺ 738.3233, found 738.3229

### (7S,9S)-9-[2-(1,4-diazepan-1-yl)acetyl]-6,9,11-trihydroxy-4-methoxy-7-[[(2S,4R,6S,7S,9R,10S)-10-methoxy-6-methyl-5,8,11-trioxa-1-azatricyclo[7.4.0.02,7]tridecan-4-yl]oxy]-8,10-dihydro-7H-tetracene-5,12-dione (II) (compound 23; X = O, A = (IIIa"))

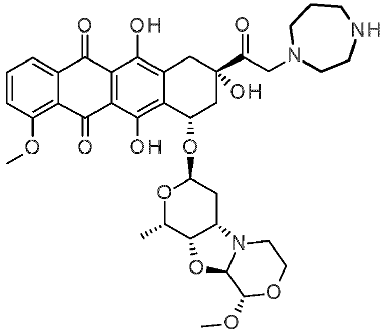

Red solid (0.015 g, 0.02 mmol, 81% yield)
HRMS (ESI) calcd for C₃₇H₄₅N₃O₁₂ [M + H]⁺ 724.3076, found 724.3073

### Step 4

### [4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate - Comp 1 (formula (I); X = NH, A = (IIIb') wherein R is a methyl group, L = (IVa) wherein r is 5)

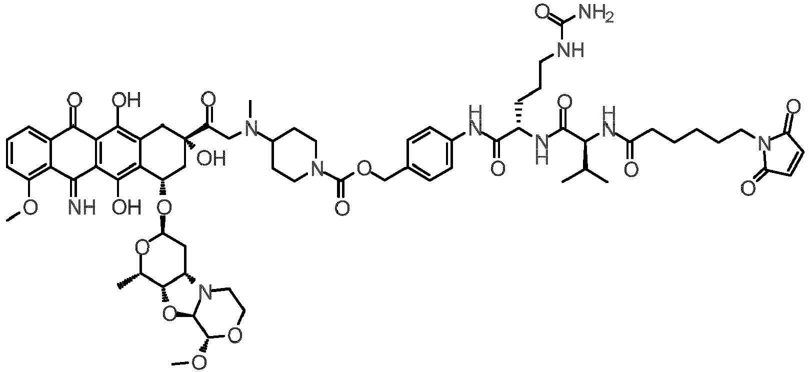

To a solution of (8S,10S)-6,8,11-trihydroxy-12-imino-1-methoxy-10-[[(2S,4R,6S,7S,9R,10S)-10-methoxy-6-methyl-5,8,11-trioxa-1-azatricyclo[7.4.0.02,7]tridecan-4-yl]oxy]-8-[2-[methyl(4-piperidyl)amino]acetyl]-9,10-dihydro-7H-tetracen-5-one (0.0038 g, 0.0051 mmol, 1 eq.) in DMF (1 mL), [4-[[(2S)-2-[[(2S)-2-[6-(2,5-dioxopyrrol-1-yl)hexanoylamino]-3-methyl-butanoyl]amino]-5-ureido-pentanoyl]amino]phenyl]methyl (4-nitrophenyl) carbonate (0.0038 g, 0.0051 mmol, 1 eq.) and TEA (0.0026 g, 0.0256 mmol, 0.0036 mL, 5 eq.) were added. The reaction mixture was stirred overnight at room temperature. Solvent was evaporated to dryness by rotovapor using an high vacuum pump. The crude was purified by chromatographic column DCM/MeOH (98/2 to 9/1) to give the title compound as a blue solid (0.0065 g, 0.0067 mmol, 38% yield).
HRMS (ESI) calcd for C₆₇H₈₆N₁₀O₁₉ [M + H]⁺ 1335.6144, found 1335.6139

Operating in an analogous way, but employing suitable substituted starting materials (intermediates XI and XII) the following compounds were obtained:

### [4-[[(2S)-2-[[(2S)-2-[6-(2,5-dioxopyrrol-1-yl)hexanoylamino]-3-methyl-butanoyl]amino]propanoyl]amino] phenyl]methyl 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate - Comp 2 (formula (I); X = NH, A = (IIIb') wherein R is a methyl group, L = (IVb) wherein r is 5)

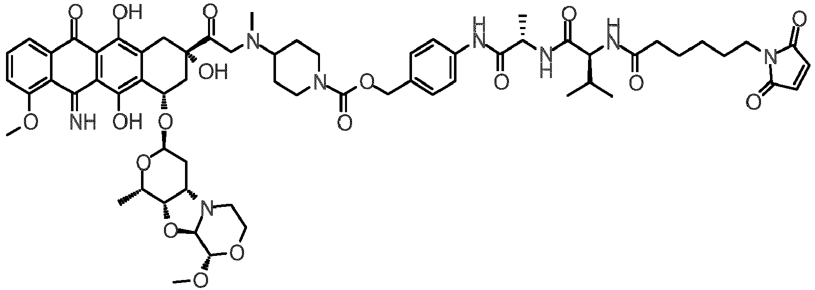

Blue solid (0.002 g, 0.0016 mmol, 34% yield).
¹H NMR (600 MHz, DMSO-*d6*) δ ppm 0.80 - 0.83 (m, 3 H) 0.83 - 0.90 (m, 3 H) 1.14 - 1-39 (m, 6 H)1.30 (d, J=7.04 Hz, 3 H) 1.44 - 1.72 (m ,7H) 1.95 (dd, J=13.35, 6.58 Hz, 1 H) 2.06 - 2.20 (m, 4 H) 2.57 - 2.69 (m, 2 H) 2.84 (d, J=18.0 Hz, 1 H) 2.94 (d, J=18.0 Hz, 1 H) 3.29 - 3.38 (m, 8 H ) 3.30 (s, 3H) 3.34 - 3.39 (m, 5 H) 3.50 (m, 1 H) 3.63 - 3.69 (m, 1 H) 3.75 (m, 2 H) 3.87 - 4.03 (m, 2 H) 3.93 (dd, J=6.49 , 1.62Hz, 1 H) 4.15 (m, 2 H) 4.12 (m, 3 H) 4.24 (m, 1 H) 4.34 - 4.43 (m, 1 H) 4.57 - 4.63 (m, 1 H) 4.91 - 5.05 (m, 3 H) 5.33 (s, 1 H) 5.37 - 5.43 (m, 1 H) 6.94 - 7.03 (m, 2 H) 7.25 - 7.32 (m, 2 H) 7.58 (d, J=8.45 Hz, 2 H) 7.64 (d, J=8.40 Hz, 1 H) 7.80 (d, J=8.67 Hz, 1 H) 7.82 - 7.91 (m, 1 H) 8.08 (d, J=7.58 Hz, 1 H) 8.15 (d, J=6.95 Hz, 1 H) 9.52 - 9.72 (m, 1 H) 9.94 (s, 1 H) 13.59 (br. s., 1 H) 15.62 - 16.06 (m, 1 H)
HRMS (ESI) calcd for C₆₄H₈₀N₈O₁₈ [M + H]⁺ 1249.5663, found 1249.5658

### [4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]piperazine-1-carboxylate - Comp 3 (formula (I); X = NH, A = (IIIa'), L = (IVa) wherein r is 5)

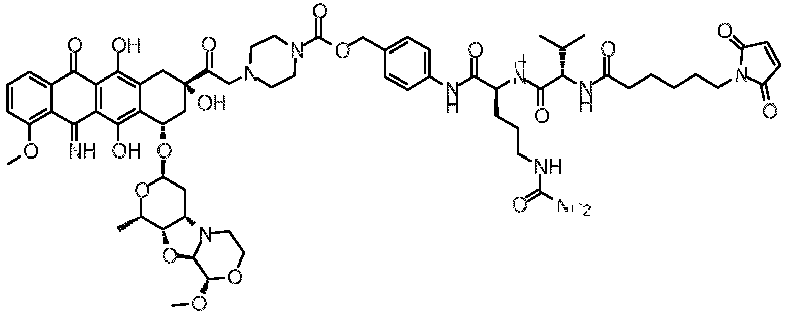

Blue solid (0.0037 g, 0.0028 mmol, 40% yield).
¹H NMR (600 MHz, DMSO-*d*₆) δ ppm 0.82 (d, *J*=6.82 Hz, 3 H) 0.85 (d, *J*=6.82 Hz, 3 H) 1.12 - 1.20 (m, 2 H) 1.23 (d, =6.38 Hz, 3 H) 1.30 - 1.53 (m, 6 H) 1.54 - 1.65 (m, 3 H) 1.65 - 1.74 (m, 1 H) 1.96 (m, 1 H) 2.03 - 2.22 (m, 4 H) 2.40 (br. s., 4 H) 2.62 (m, 1 H) 2.67 (m, 1 H) 2.85 (d, *J*=18.00, 1 H) 2.90 (d, *J*=18.00, 1 H) 2.91 - 2.97 (m, 1 H) 3.03(m, 1 H) 3.30 (s, 3 H) 3.33 - 3.42 (m, 7 H) 3.50 (m, 1 H) 3.61 - 3.72 (m, 3 H) 3.93 (m, 1 H) 4.12 (s, 3 H) 4.14 (m., 1 H) 4.19 (dd, J=8.58, 7.26, 1 H) 4.23 (d, *J*=1.98 Hz, 1 H) 4.38 (m, 1 H) 4.60 (d, *J*=1.76 Hz, 1 H) 4.99 (s, 2 H) 5.05 (t, *J*=3.52 Hz, 1 H) 5.34 (s, 1 H) 5.40 (s, 3 H) 5.96 (t, *J*=5.72 Hz, 1 H) 7.00 (s, 2 H) 7.28 (d, *J*=8.58 Hz, 2 H) 7.59 (d, *J*=8.58 Hz, 2 H) 7.64 (d, *J*=8.36 Hz, 1 H) 7.80 (d, *J*=8.80 Hz, 1 H) 7.87 (t, *J*=7.92 Hz, 1 H) 8.08 (m, 2 H) 9.66 (d, *J*=5.00 Hz 1 H) 9.99 (s, 1 H) 13.59 (d, *J*=5.00 Hz, 1 H) 15.88 (s, 1 H)
HRMS (ESI) calcd for C₆₅H₈₂N₁₀O₁₉ [M + H]⁺ 1307.5831, found 1307.5836

### [4-[[(2S)-2-[[(2S)-2-[6-(2,5-dioxopyrrol-1-yl)hexanoylamino]-3-methyl-butanoyl]amino]propanoyl] amino] phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]piperazine-1-carboxylate - Comp 4 (formula (I); X = NH, A = (IIIa'), L = (IVb) wherein r is 5)

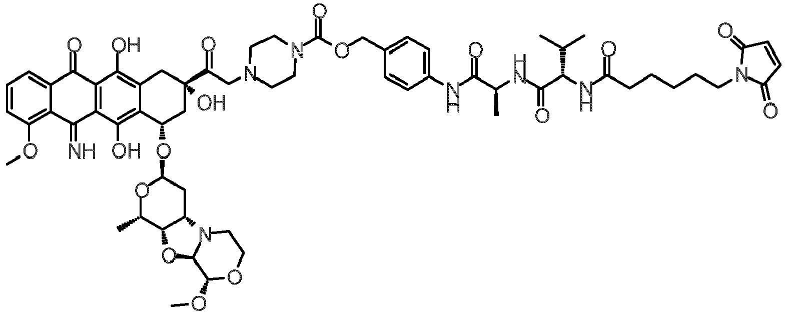

Blue solid (0.0075 g, 0.0061 mmol, 73% yield).
¹H NMR (500 MHz, DMSO-*d*₆) δ ppm 0.82 (d, *J*=6.70 3 H) 0.86 (d, *J*=6.70 3 H)1.12 - 1.26 (m, 5 H) 1.30 (d, *J*=7.13 Hz, 3 H) 1.39 - 1.52 (m, 4 H) 1.55 - 1.68 (m, 2 H) 1.90 - 2.22 (m, 5 H) 2.40 (br. s., 3 H) 2.57 - 2.72 (m, 3 H) 2.81 - 2.95 (m, 2 H) 3.30 (s, 4 H) 3.33 - 3.40 (m, 7 H) 3.45 - 3.55 (m, 1 H) 3.63 - 3.75 (m, 3 H) 3.93 (d, *J*=7.26 Hz, 1 H) 4.12 (s, 3 H) 4.14 - 4.20 (m, 1 H) 4.23 (d, *J*=1.56 Hz, 1 H) 4.33 - 4.42 (m, 1 H) 4.59 (s, 1 H) 4.99 (s, 2 H) 5.02 - 5.07 (m, 1 H) 5.35 (br. s., 1 H) 5.40 (t, *J*=4.48 Hz, 1 H) 7.00 (s, 2 H) 7.29 (d, *J*=8.43 Hz, 2 H) 7.58 (d, *J*=8.43 Hz, 2 H) 7.64 (d, *J*=6.36 Hz, 1 H) 7.82 (d, *J*=8.56 Hz, 1 H) 7.86 (m., 1 H) 8.08 (d, *J*=7.91 Hz, 1 H) 8.18 (d, *J*=6.88 Hz, 1 H) 9.67 (br. s., 1 H) 9.96 (s, 1 H) 13.59 (br. s., 1 H) 15.88 (br. s., 1 H)
HRMS (ESI) calcd for C₆₂H₇₆N₈O₁₈ [M + H]⁺ 1221.5351, found 1221.5363

### [4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl N-methyl-N-[1-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-4-piperidyl]carbamate - Comp 5 (formula (I); X = NH, A = (IIIc') wherein R is a methyl group, L = (IVa) wherein r is 5)

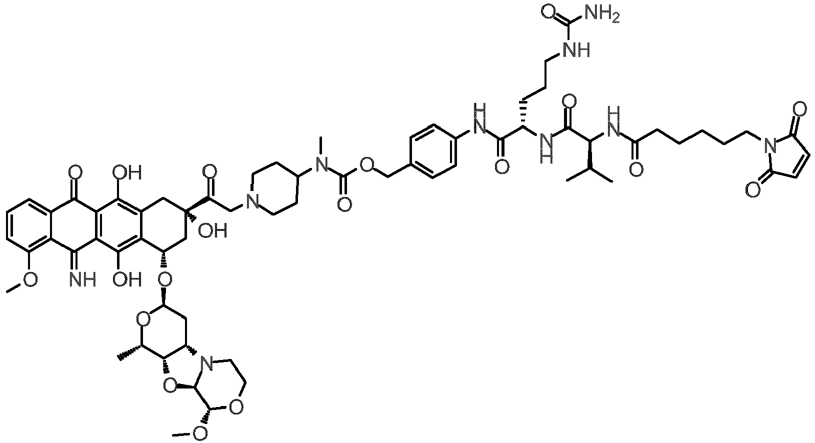

Blue solid (0.0065 g, 0.0049 mmol, 41% yield).
HRMS (ESI) calcd for C₆₇H₈₆N₁₀O₁₉ [M + H]⁺ 1335.6144, found 1335.6151

### [4-[[(2S)-2-[[(2S)-2-[6-(2,5-dioxopyrrol-1-yl)hexanoylamino]-3-methyl-butanoyl]amino]propanoyl]amino] phenyl]methyl N-methyl-N-[1-[2-oxo-2-[(2S,4S)-2,S,12-trihydroxy-6-imino-7-methoxy-4-[[(2S,4R,6S,7S,9R,10S)-1 0-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-4-piperidyl]carbamate - Comp 6 (formula (I); X = NH, A = (IIIc') wherein R is a methyl group, L = (IVb) wherein r is 5)

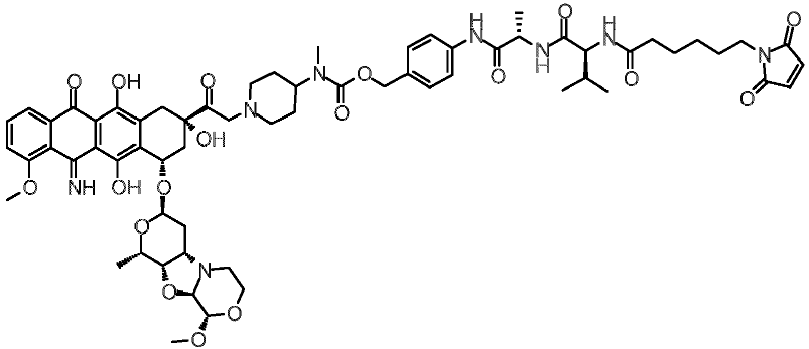

Blue solid (0.012 g, 0.0096 mmol, 28% yield).
HRMS (ESI) calcd for C₆₄H₈₀N₈O₁₈ [M + H]⁺ 1249.5663, found 1249.5670

### [4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl 4-[methyl-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]amino]piperidine-1-carboxylate - Comp 7 (formula (I); X = O, A = (IIIb') wherein R is a methyl group, L = (IVa) wherein r is 5)

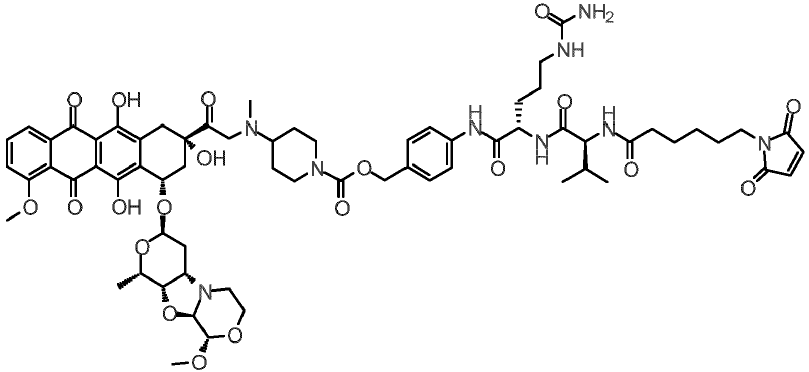

Red solid (0.0027 g, 0.002 mmol, 43% yield).
HRMS (ESI) calcd for C₆₇H₈₅N₉O₂₀ [M + H]⁺ 1336.5984, found 1336.5991

### [4-[[(2S)-2-[[(2S)-2-[6-(2,5-dioxopyrrol-1-yl)hexanoylamino]-3-methyl-butanoyl]amino]propanoyl] amino] phenyl]methyl 4-[methyl-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]amino]piperidine-1-carboxylate - Comp 8 (formula (I); X = O, A = (IIIb') wherein R is a methyl group, L = (IVb) wherein r is 5)

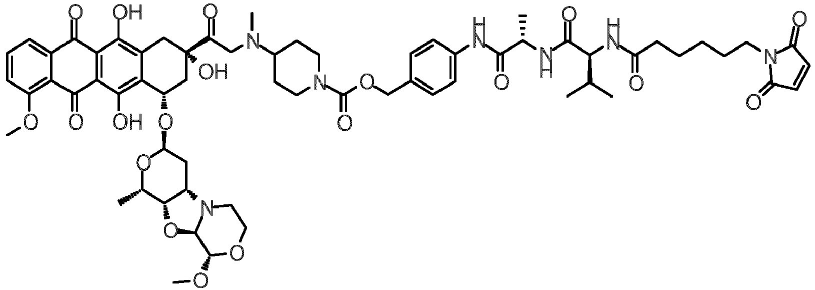

Red solid (0.0048 g, 0.0038 mmol, 46% yield).
HRMS (ESI) calcd for C₆₄H₇₉N₇O₁₉ [M + H]⁺ 1250.5504, found 1250.5510

### [4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]piperazine-1-carboxylate - Comp 9 (formula (I); X = O, A = (IIIa'), L = (IVa) wherein r is 5)

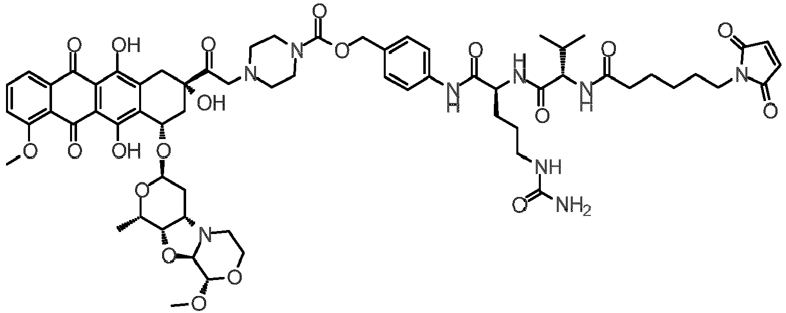

Red solid (0.0044 g, 0.0034 mmol, 64% yield).
HRMS (ESI) calcd for C₆₅H₈₁N₉O₂₀ [M + H]⁺ 1308.5671, found 1308.5679

### [4-[[(2S)-2-[[(2S)-2-[6-(2,5-dioxopyrrol-1-yl)hexanoylamino]-3-methyl-butanoyl]amino]propanoyl] amino] phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]piperazine-1-carboxylate - Comp 10 (formula (I); X = O, A = (IIIa'), L = (IVb) wherein r is 5)

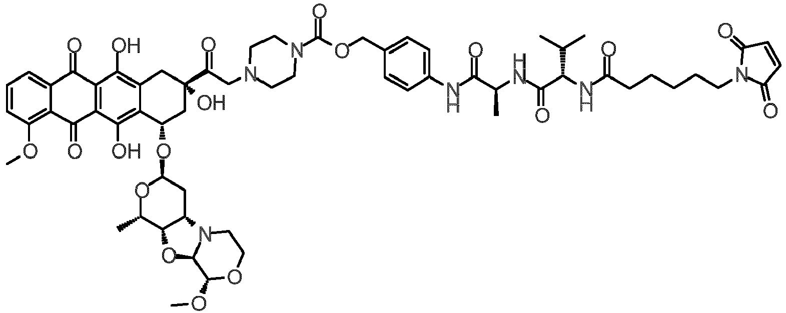

Red solid (0.0085 g, 0.007 mmol, 62% yield).
¹H NMR (500 MHz, DMSO-*d6*) δ ppm 0.78 - 0.88 (m, 6 H) 1.13 - 1.20 (m, 2 H) 1.23 (m, 3H) 1.30 (d, *J*=7.08 Hz, 3 H) 1.41 - 1.53 (m, 4 H) 1.66 (br. s., 2 H) 1.91 - 2.03 (m, 1 H) 2.07 - 2.23 (m, 4 H) 2.39 (br. s., 4 H) 2.57 - 2.72 (m, 2 H) 2.95 (q, *J*=17.90 Hz, 2 H) 3.30 (s, 3 H) 3.31 - 3.40 (m, 7H) 3.46 - 3.54 (m, 1 H) 3.60 - 3.73 (m, 3 H) 3.93 (d, *J*=7.45 Hz, 1 H) 3.99 (s, 3 H) 4.14 - 4.20 (m, 2 H) 4.23 (d, *J*=1.83 Hz, 1 H) 4.38 (t, *J*=7.08 Hz, 1 H) 4.59 (d, *J*=1.71 Hz, 1 H) 4.99 (s, 3 H) 5.22 (t, *J*=4.21 Hz, 1 H) 5.45 (s, 1 H) 7.00 (s, 2 H) 7.29 (d, *J*=8.55 Hz, 2 H) 7.58 (d, *J*=8.42 Hz, 2 H) 7.66 (d, *J*=6.23 Hz, 1 H) 7.81 (d, *J*=8.67 Hz, 1 H) 7.89 - 7.96 (m, 2 H) 8.17 (d, *J*=6.84 Hz, 1 H) 9.95 (s, 1 H) 13.13 - 13.42 (m, 1 H) 13.89 - 14.18 (m, 1 H)
HRMS (ESI) calcd for C₆₂H₇₅N₇O₁₉ [M + H]⁺ 1222.5191, found 1222.5194

### [4-[[(2S)-2-[[(2S)-2-[6-(2,5-dioxopyrrol-1-yl)hexanoylamino]-3-methyl-butanoyl]amino]-5-ureido-pentanoyl] amino]phenyl]methyl N-methyl-N-[1-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl]carbamate - Comp 11 (formula (I); X = O, A = (IIIc') wherein R is a methyl group, L = (IVa) wherein r is 5)

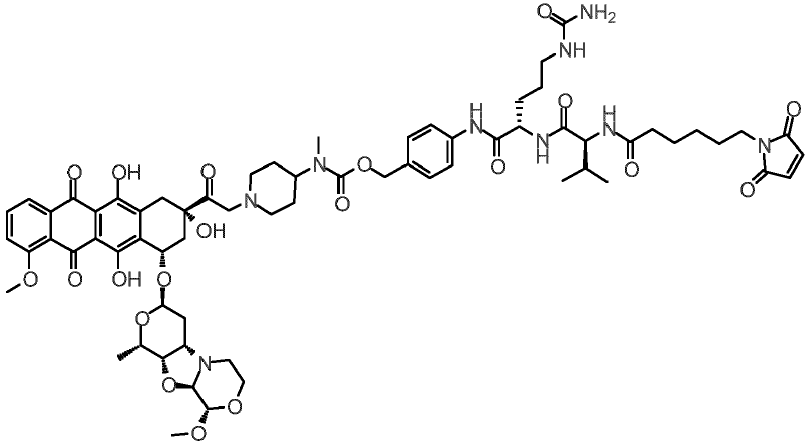

Red solid (0.0075 g, 0.0056 mmol, 44% yield).
HRMS (ESI) calcd for C₆₇H₈₅N₉O₂₀ [M + H]⁺ 1336.5984, found 1336.5979

### [4-[[(2S)-2-[[(2S)-2-[6-(2,5-dioxopyrrol-1-yl)hexanoylamino]-3-methyl-butanoyl]amino]propanoyl] amino]phenyl]methyl N-methyl-N-[1-[2-oxo-2-[(2S,4S)-2,5,12-trihydroxy-7-methoxy-4-[[(2S,4R,6S,7S,9R,10S)-1 0-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl]carbamate - Comp 12 (formula (I); X = O, A = (IIIc') wherein R is a methyl group, L = (IVb) wherein r is 5)

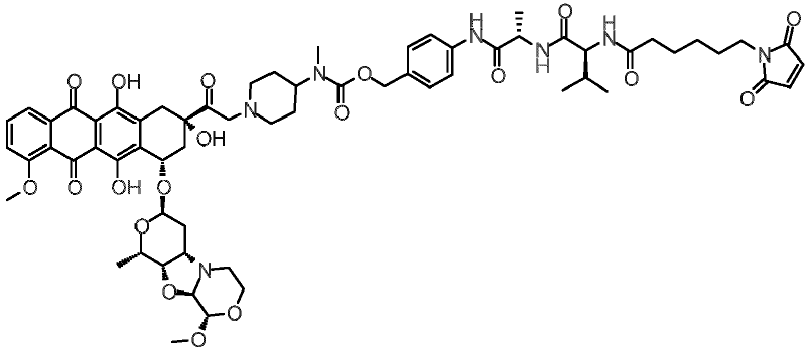

Red solid (0.0043 g, 0.0034 mmol, 28% yield).
1H NMR (600 MHz, DMSO-d6) δ ppm 0.77 - 0.91 (m, 6 H) 0.80 (m, 2H) 1.23 (d, J =6.54, 3H) 1.30 (d, J=7.27 Hz, 3 H) 1.40 - 1.52 (m, 6 H) 1.64 - 1.69 (m, 4 H) 1.95 (m, 1 H) 2.02 - 2.24 (m, 6 H) 2.69 - 2.68 (m, 2 H) 2.70 (s, 3 H) 2.83 (m, 2 H) 2.90 (d, J=17.98 Hz, 1 H) 3.02 (d, J=17.98 Hz, 1 H) 3.30 (s, 3 H) 3.34 - 3.39 (m, 3 H) 3.50 (m, 1 H) 3.55 - 3.64 (m, 2 H) 3.66 (m, 1 H) 3.93 (m, 1 H) 3.99 (s, 3 H) 4.16 (dd, J=8.63, 6.99 Hz, 2 H) 4.23 (d, J=2.00 Hz, 1 H) 4.38 (m, 2 H) 4.59 (d, J=2.00 Hz, 1 H) 4.90 - 5.04 (m, 3 H) 5.23 (t, J=4.54 Hz, 1 H) 5.47 (s, 1 H) 6.99 (s, 2 H) 7.22 - 7.34 (m, 2 H) 7.54 - 7.61 (m, 2 H) 7.67 (m, 1 H) 7.80 (d, J=8.72 Hz, 1 H) 7.87.95 (m, 2 H) 8.15 (d, J=7.08 Hz, 1 H) 9.93 (s, 1 H) 13.28 (br. s., 1 H) 14.05 (br. s., 1 H)
HRMS (ESI) calcd for C₆₄H₇₉N₇O₁₉ [M + H]⁺ 1250.5504, found 1250.5507

### [4-[[(2S)-2-[[(2S)-2-[6-(2,5-dioxopyrrol-1-yl)hexanoylamino]-3-methyl-butanoyl]amino]propanoyl] amino] phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-1,4-diazepane-1-carboxylate - Comp 16 (formula (I); X = O, A = (IIIa"), L = (IVb) wherein r is 5)

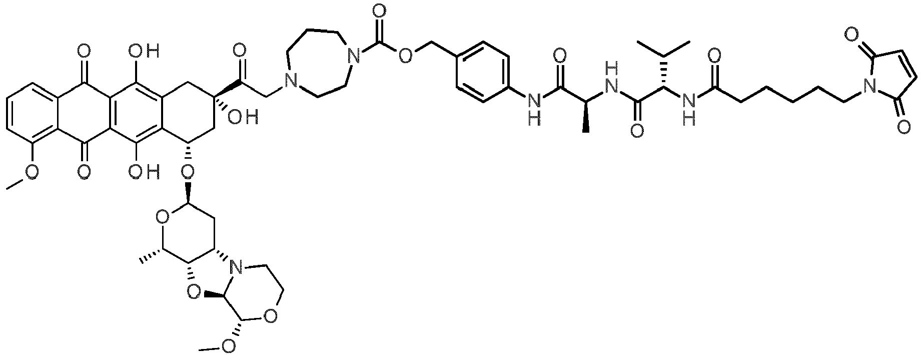

Red solid (0.0081 g, 0.0065 mmol, 61% yield).
HRMS (ESI) calcd for C₆₃H₇₇N₇O₁₉ [M + H]⁺ 1236.5347, found 1236.5351

## Claims

1. An anthracycline-linker reagent of formula (I):
Ant- L (I)
wherein:
**Ant** is an anthracycline drug moiety of formula (II): 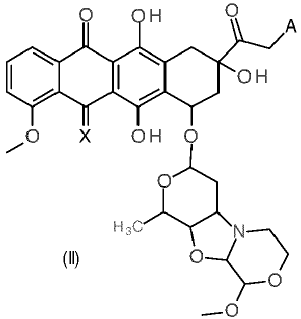 wherein:
**X** is O or NH and
**A** is a heterocycle (Het) or carbocycle (Cb) selected from the group consisting of (IIIa)-(IIIe): 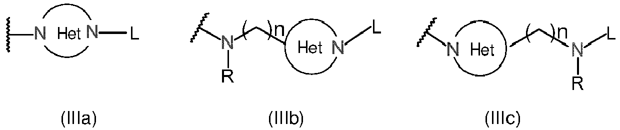 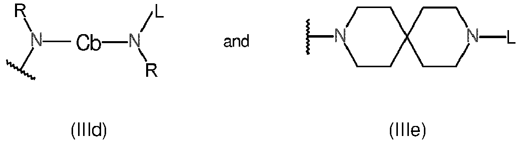 wherein:
**R** is independently hydrogen, or a straight or branched (C₁-C₆) alkyl;
**n** is an integer from 0 to 6;
**L** is a linker of formula (VI):
W-PE-RM (VI)
wherein:
**W** is independently null or a self immolative system selected from the group consisting of (Va)-(Vc): 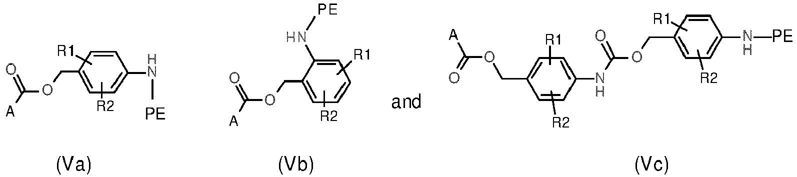 wherein:
**R1** and **R2** are, each independently, hydrogen, halogen, methyl, ethyl or straight or branched C₁-C₄ hydroxyalkyl;
**PE** is independently null or a dipeptidic or tripeptidic moiety, consisting of any combination of natural L-aminoacids and unnatural D-aminoacids.
**RM** is a reactive moiety selected from the group consisting of (Vla)-(Vlc): 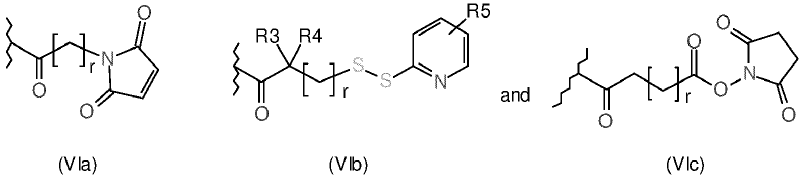 wherein:
**R3** and **R4** are, each independently, hydrogen, halogen, methyl, ethyl, straight or branched C₁-C₄ hydroxyalkyl, straight or branched C₁-C₄ haloalkyl, or R3 and R4 taken together form a 3- to 6-membered carbocycle;
**R5** is hydrogen, C₁-C₃ alkyl or an electron-withdrawing group, comprising NO₂ or CN group;
**r** is an integer from 0 to 7.

2. A compound of the formula (I) according to claim 1 wherein:
**A** is an optionally heterocyclic or carbocyclic group selected from the group consisting of: 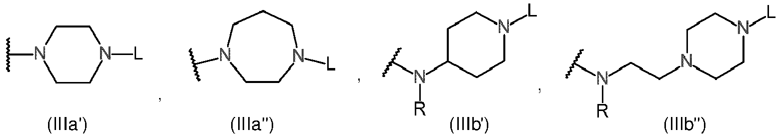 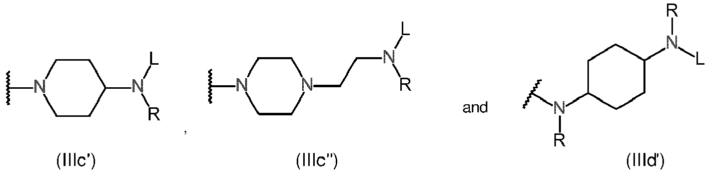 wherein:
**R** is a straight or branched (C₁-C₆) alkyl;
L is a linker of formula (VI) wherein **W** is a group (Va') or (Vb'): 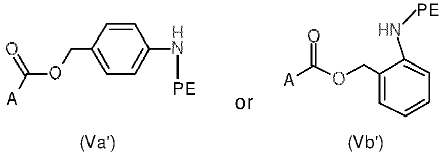
**PE** is a dipeptide or tripeptide moiety, consisting of any combination of the following aminoacids: glycine, alanine, leucine, valine, citrulline, phenylalanine, wherein the C-terminal aminoacid residue is linked to W, and the N-terminal aminoacid residue is linked to **RM;**
**RM** is a group selected from (Vla)-(Vlc): 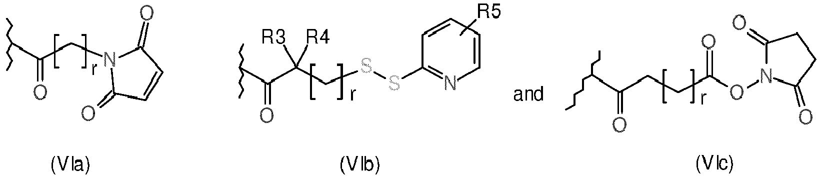 wherein:
**R3, R4** and **R5** are hydrogen;
**r** is an integer preferably from 3 to 5.

3. A compound of the formula (I) according to claim 2 wherein:
**A** is a heterocyclic group selected from the group consisting of (IIIa'), (IIIa"), (IIIb') and (IIIc');
**W** is a group (Va') or (Vb'):
**PE** is a dipeptide selected from valine-alanine and valine-citrulline or a tripeptide phenylalanine-leucine-glycine wherein the C-terminal aminoacid residue is linked to **W** and the N-terminal aminoacid residue is linked to **RM;**
**RM** is a reactive moiety selected from the group (Vla)-(Vlc).

4. A compound of the formula (I) according to claim 3 wherein:
**A** is a heterocyclic group selected from the group consisting of (IIIa'), (IIIa"), (IIIb') and (IIIc'); and
**L** is a linker selected from the group consisting of (IVa)-(IVc): 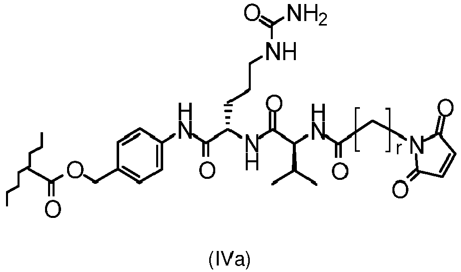 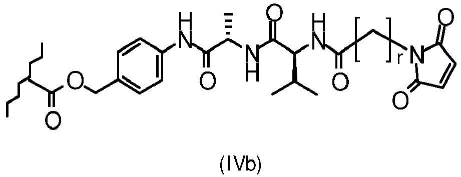 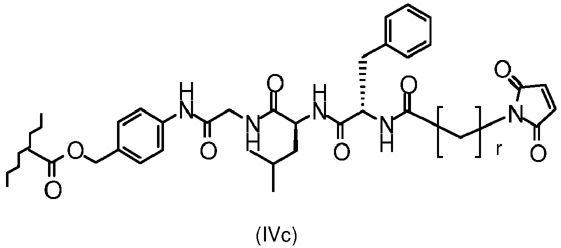 wherein:
**r** is an integer from 3 to 5.

5. A compound (comp) of formula (I), according to claim 1, selected from the group consisting of:
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate (comp 1);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[methyl-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]amino]piperidine-1-carboxylate (comp 2);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]piperazine-1-carboxylate (comp 3);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]piperazine-1-carboxylate (comp 4);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl N-methyl-N-[1-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7jtridecan-4-yl]oxy]-11-oxo-3,4-dihydro-1H-tetracen-2-yl]ethyl]-4-piperidyl]carbamate (comp 5);
[4-[[(2S)-2-[[(2S)-2-[6-(2,5-dioxopyrrol-1-yl)hexanoylamino]-3-methyl-butanoyl]amino]propanoyl]amino]phenyl]methyl N-methyl-N-[1-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-4-piperidyl] carbamate (comp 6);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[methyl-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl] aminojpiperidine-1-carboxylate (comp 7);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[methyl-[2-oxo-2-[(2S,4S)-2,5,12-trihydroxy-7-methoxy-4-[[(2S,4R,6S,7S,9R,10S)-10-methoxy-6-methyl-5,8,11 - trioxa-1-azatricyclo[7.4.0.02,7]tridecan-4-yl]oxy]-6,11-dioxo-3,4-dihydro-1H-tetracen-2-yl]ethyl]amino]piperidine-1-carboxylate (comp 8);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]piperazine-1-carboxylate (comp 9);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]piperazine-1-carboxylate (comp 10);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl N-methyl-N-[1-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl]carbamate (comp 11);
[4-[[(2S)-2-[[(2S)-2-[6-(2,5-dioxopyrrol-1-yl)hexanoylamino]-3-methyl-butanoyl]amino]propanoyl]amino]phenyl]methyl N-methyl-N-[1-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-4-piperidyl] carbamate (comp 12);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-1,4-diazepane-1-carboxylate (comp 13);
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-6-imino-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-11-oxo-3,4-dihydro-1H-tetracen-2-yl]ethyl]-1,4-diazepane-1-carboxylate (comp 14);
[4-[[(2S)-2-[[(2S)-2-[6-(2,5-dioxopyrrol-1-yl)hexanoylamino]-3-methyl-butanoyl]amino]-5-ureido-pentanoyl]amino] phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-1,4-diazepane-1-carboxylate (comp 15) and
[4-[[(2S)-2-[[(2S)-2-[6-(2,5-dioxopyrrol-1-yl)hexanoylamino]-3-methyl-butanoyl]amino]propanoyl]amino]phenyl]methyl 4-[2-oxo-2-[(2S,4S)-2,5,12-trihydroxy-7-methoxy-4-[[(2S,4R,6S,7S,9R,10S)-10-methoxy-6-methyl-5,8,11-trioxa-1-azatricyclo[7.4.0.02,7]tridecan-4-yl]oxy]-6,11-dioxo-3,4-dihydro-1H-tetracen-2-yl]ethyl]-1,4-diazepane-1-carboxylate (comp 16).

6. Antibody-drug conjugates (ADCs) of formula (Ia): 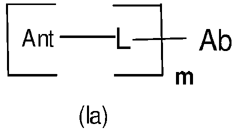 wherein:
**Ab** includes any unit, type, or class of antibody that binds or reactively associates or complexes with a receptor, antigen or other receptive moiety associated with a given target-cell population;
**m** (drug loading) is 1, 2, 3, 4, 5, 6, 7 or 8; and
**Ant-L** is an anthracycline-linker reagent of formula (I) according to any of claims 1-5.

7. Antibody-drug conjugates (ADCs) of formula (Ia) as defined in claim 6 wherein **Ant-L** is a compound of claim 5.

8. Antibody-drug conjugates (ADCs) of formula (Ia) as defined in claim 6 wherein **Ab** is an antibody which binds to one or more tumor-associated ErbB2 antigens or cell-surface ErbB2 receptor.

9. Antibody-drug conjugates (ADCs) of formula (Ia) according to claim 6 selected from the group consisting of compounds (Ia^{I}) - (Ia^{XVI}): 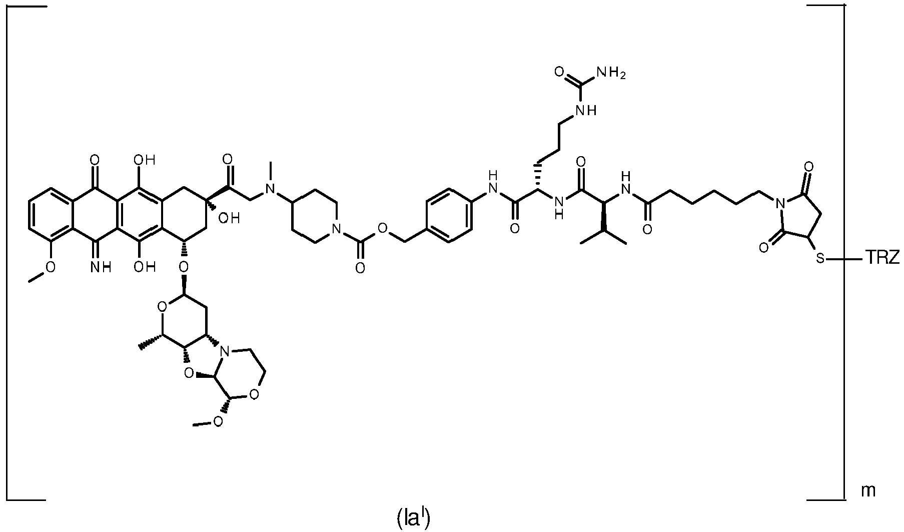 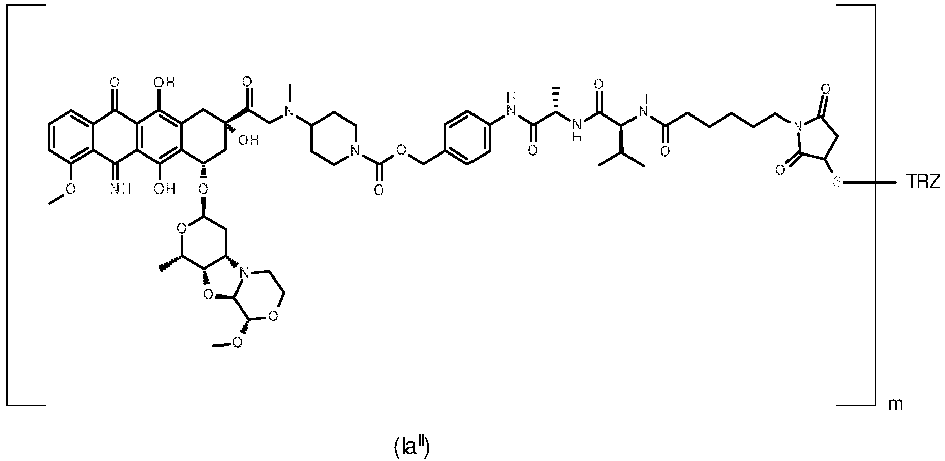 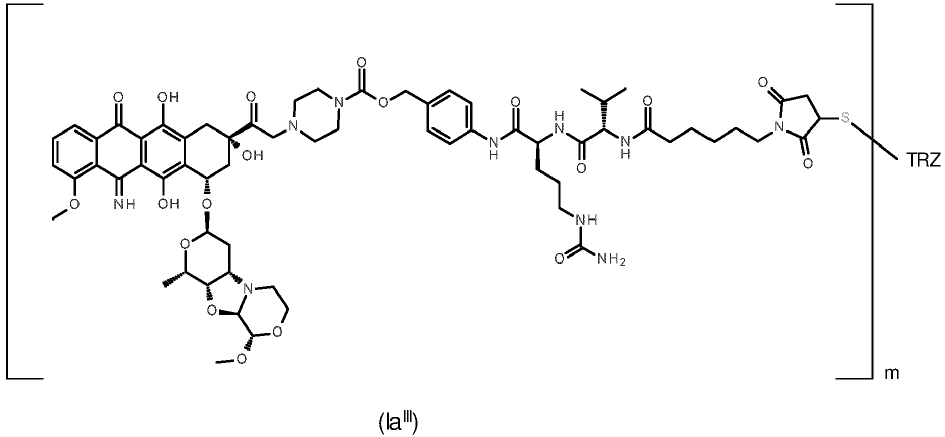 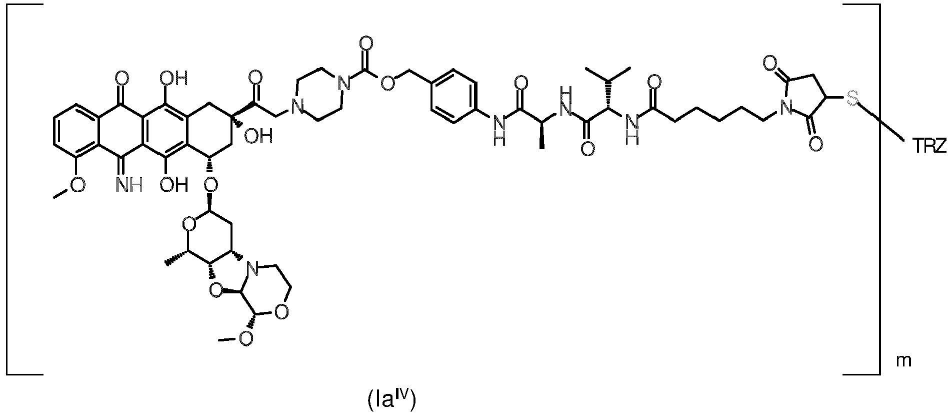 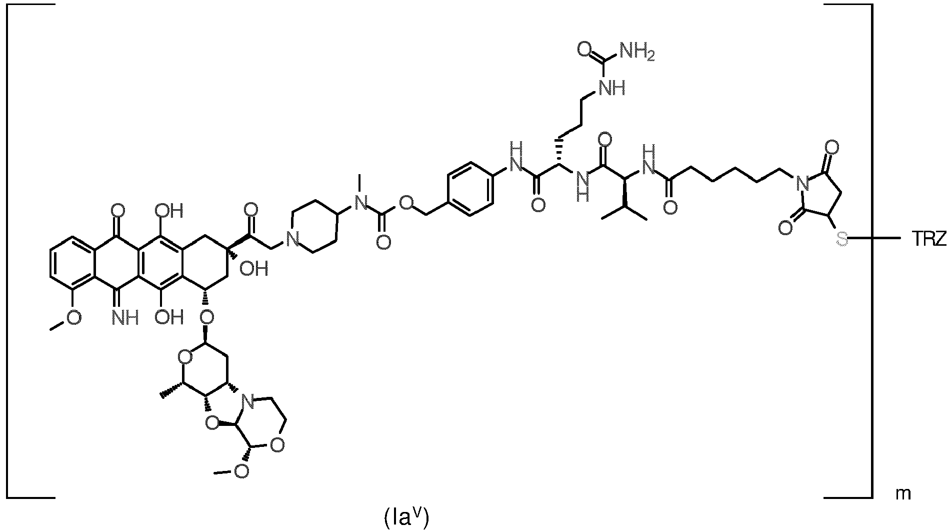 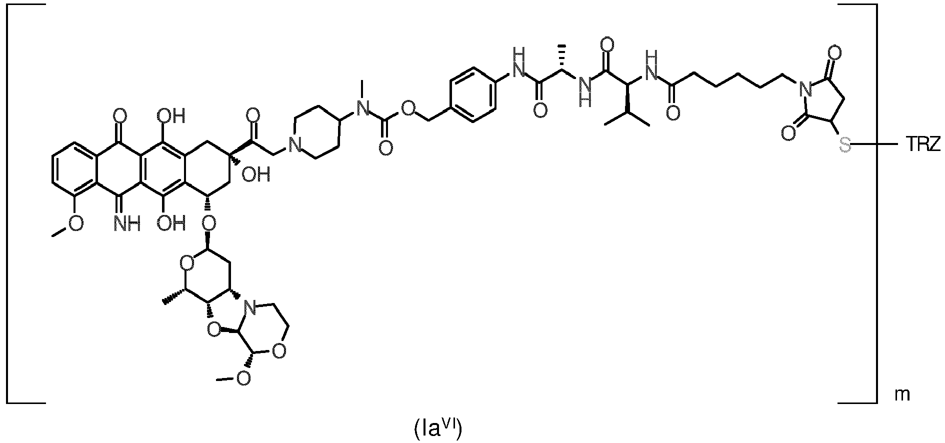 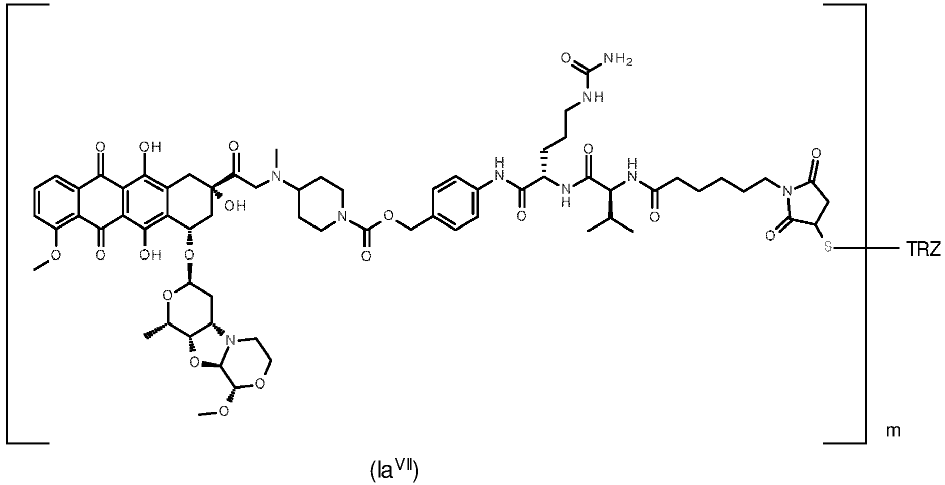 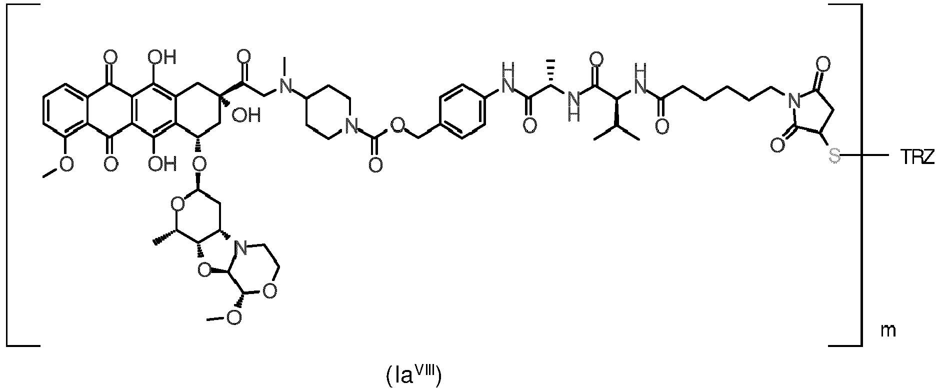 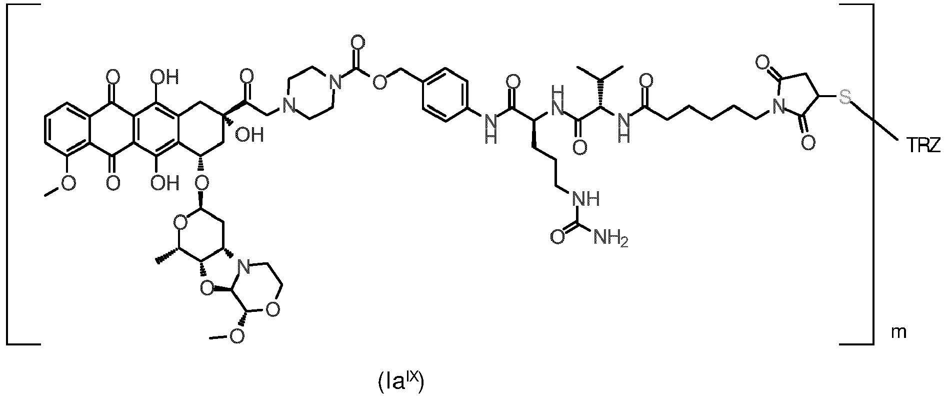 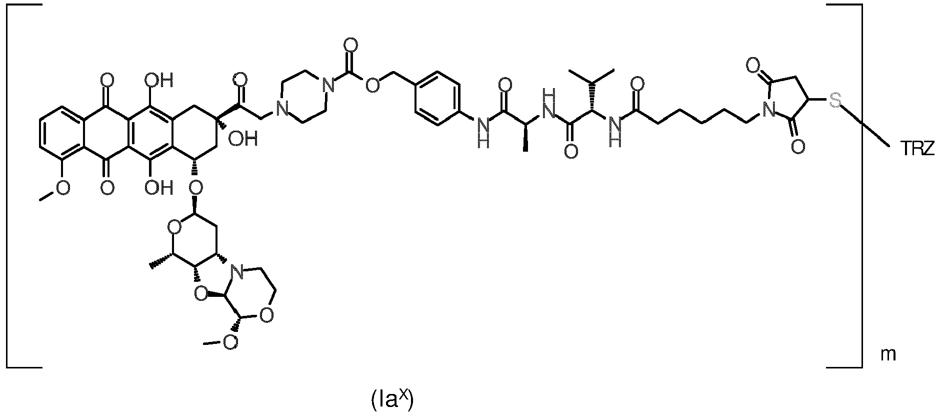 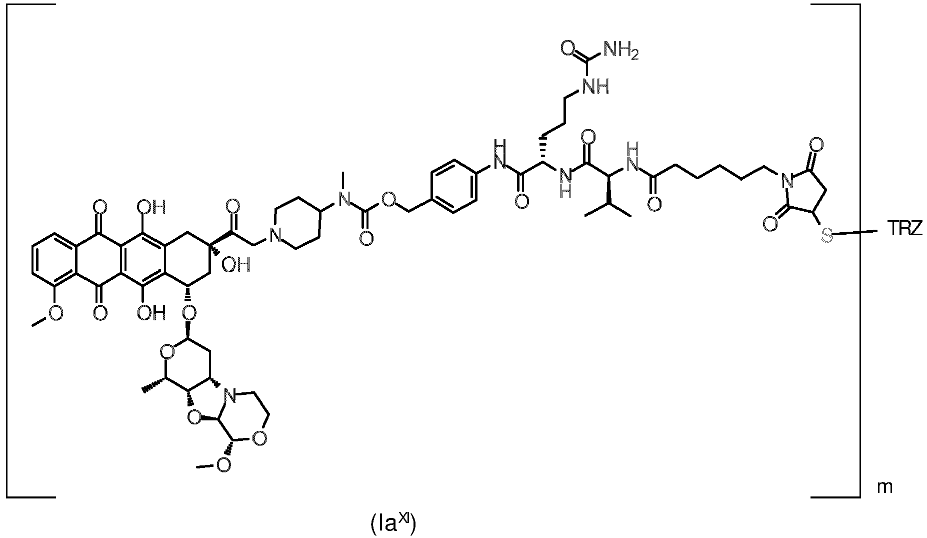 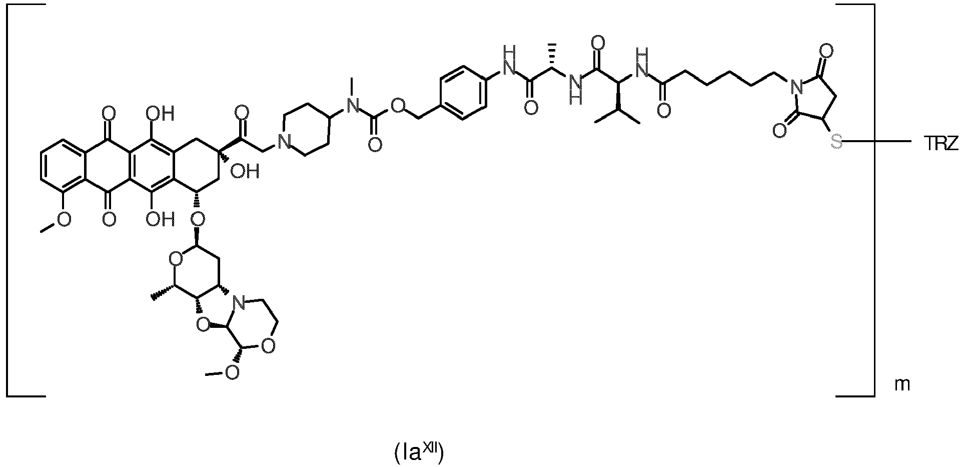 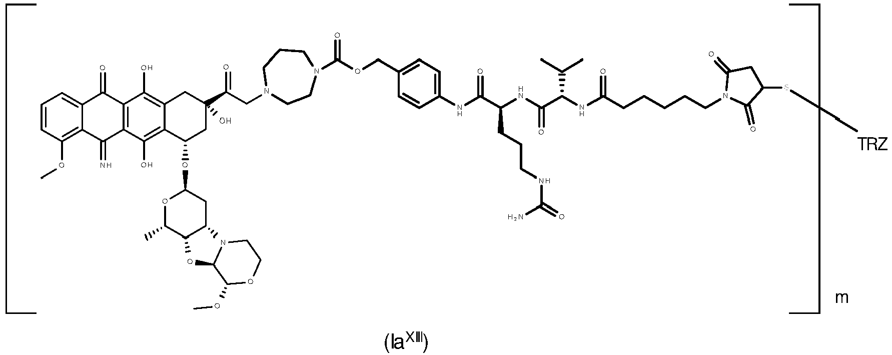 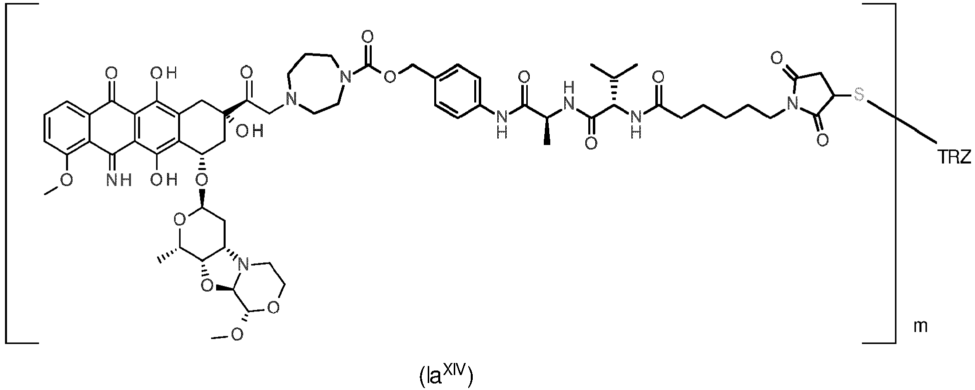 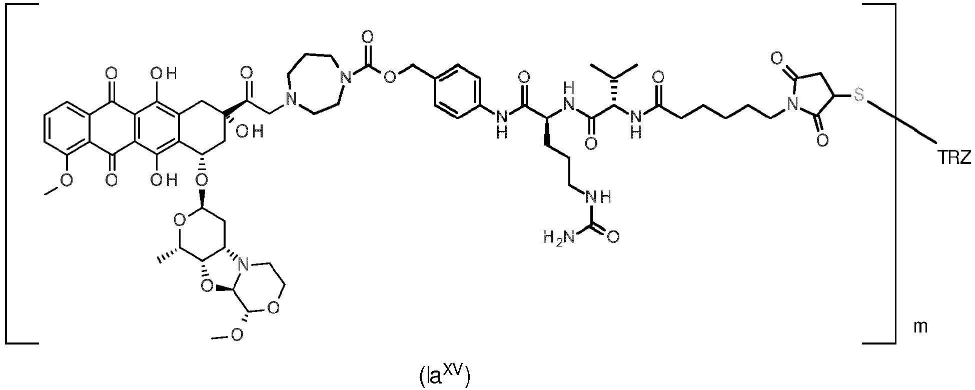 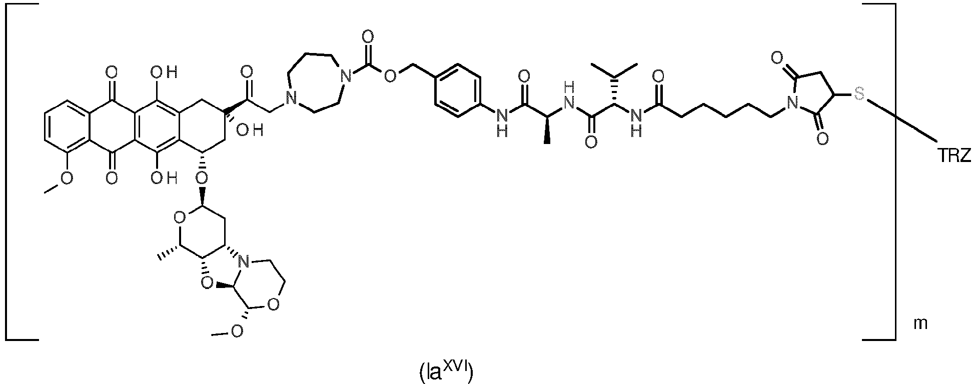 wherein m is 1, 2, 3 or 4 and TRZ is Trastuzumab.

10. A pharmaceutical composition comprising a mixture of antibody-drug conjugate compounds of formula (Ia) or a pharmaceutically acceptable salt thereof, as defined in claim 6, wherein the average drug loading per antibody in the mixture of antibody-drug conjugate compound is about 2 to about 4.

11. An antibody-drug conjugate compound of formula (Ia) or a pharmaceutically acceptable salt thereof, as defined in claim 6, for use as a medicament.

12. Antibody-drug conjugate compounds of formula (Ia) or a pharmaceutically acceptable salt thereof, as defined in claim 6, for use in a method for treating cancer.

13. Use of anthracycline-linker reagent compounds of formula (I) as defined in claim 1, in the preparation of antibody-drug conjugate compounds of formula (Ia) as defined in claim 6.

14. The anthracycline derivatives intermediates selected from:

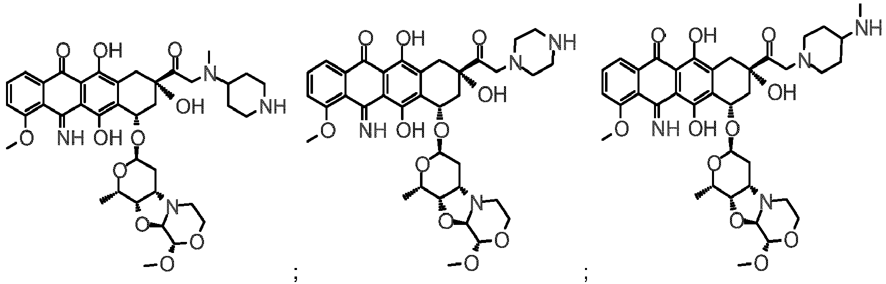

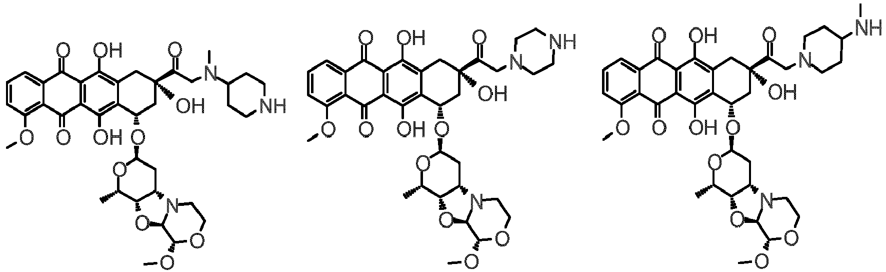

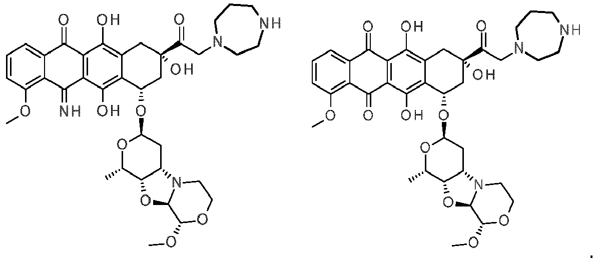

15. Use of the anthracycline derivatives intermediates according to claim 14 for the synthesis of anthracycline-linker reagent compounds of formula (I).
